(19)

**Europäisches Patentamt**
**European Patent Office**
**Office européen des brevets**

(11) **EP 4 309 512 A1**

(12) **EUROPEAN PATENT APPLICATION**

(43) Date of publication:
**24.01.2024 Bulletin 2024/04**

(21) Application number: **23187122.9**

(22) Date of filing: **21.07.2023**

(51) International Patent Classification (IPC):
**A23L 5/00** *(2016.01)* **A61K 8/31** *(2006.01)*
**A61K 36/00** *(2006.01)* **A61K 47/46** *(2006.01)*
**A61Q 19/00** *(2006.01)*

(52) Cooperative Patent Classification (CPC):
(C-Sets available)
**A23L 33/115; A61K 8/31; A61K 36/28;**
**A61K 36/63; A61K 47/46; A61Q 19/00;**
A61K 2236/333 (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **21.07.2022 IT 202200015423**

(71) Applicant: **Argiolas S.p.A.**
**09040 Serdiana (CA) (IT)**

(72) Inventors:
• **MANCONI, Maria**
**09124 Cagliari (IT)**

• **MANCA, Maria Letizia**
**09128 Cagliari (IT)**
• **CASTANGIA, Ines**
**09131 Cagliari (IT)**
• **ARGIOLAS, Francesca**
**09040 Serdiana (CA) (IT)**
• **PINNA, Maria Barbara**
**09131 Cagliari (IT)**
• **MURRU, Mariano**
**09126 Cagliari (IT)**

(74) Representative: **Primiceri, Maria Vittoria et al**
**Praxi Intellectual Property S.p.A.**
**Via Leonida Bissolati, 20**
**00187 Roma (IT)**

(54) **ONE SHOT PROCESS FOR THE SIMULTANEOUS EXTRACTION OF PHYTOCOMPLEX FROM AGRO-INDUSTRIAL PROCESSING WASTE AND LOADING IN EXTRACTIVE VESICLES AND THEIR USE**

(57) The present invention relates to a process for the treatment and valorisation of agro-food production waste such as pomace and to the products thus obtained. In particular, the invention refers to a process for the extraction of phytocomplexes from agro-industrial waste by the addition of phospholipids and the simultaneous formation of phospholipidic vesicles loaded with said phytocomplexes. The extraction and delivery take place one-shot and make it possible to obtain pomace phytocomplexes delivered in phospholipidic vesicles by simply adding the phospholipids directly into the extraction solution. The products thus obtained can be used in the pharmaceutical, cosmetic and nutraceutical fields.

EP 4 309 512 A1

(52) Cooperative Patent Classification (CPC): (Cont.)

C-Sets
**A61K 36/28, A61K 2300/00;**
**A61K 36/63, A61K 2300/00**

**Description**

Technical field

The present invention refers to a process for the treatment of agro-industrial processing waste, in particular pomace, and to the products thus obtained. In particular, the invention refers to a process for the extraction of phytocomplexes obtained from said waste, in particular pomace, by adding phospholipids and the simultaneous formation of phospholipidic vesicles carrying said phytocomplexes (called extractive vesicles).

The process consists of a one-shot extraction which allows the phytocomplexes contained in agro-industrial processing waste, such as pomace, to be conveyed inside phospholipidic vesicles, which are formed extemporaneously by adding the phospholipids directly into the extraction solution and thus forming the extractive vesicles. In this way the extraction, the loading of the phytocomplex and the formation of the vesicles are obtained simultaneously. Advantageously, the phytocomplexes contained in the vesicles are rich in both hydrophilic and lipophilic molecules.

The invention also relates to the products obtained with said one-shot process, also defined hereinafter as extractive phospholipidic vesicles or simply extractive vesicles, which can be used in the pharmaceutical, cosmetic and nutraceutical fields.

The extractive vesicles can also be freeze-dried (in the presence of a cryoprotectant) to obtain a complex solid and stable over time, and extemporaneously rehydrated to reconstitute the aforementioned vesicular dispersion, at the time of use.

Optionally, the vesicles of the present invention can further contain or be added with suitable excipients which increase their stability, protect them from the biological environment, facilitate the application of the products, and increase the local or systemic bioavailability of the carrier substances.

Background art

Reactive oxygen species (ROS) are commonly generated in the human body from environmental or endogenous causes. The ROS scavenging system is present in many tissues as a defense mechanism, but is only able to neutralize a limited number of these species. When ROS accumulate in a tissue they can react and modify proteins, unsaturated lipids and nucleic acids causing an alteration or destruction of cellular functionality and their consequent irreversible modification or death. This condition, called oxidative stress, if chronic, can cause moderate or severe health problems, such as premature aging, chronic inflammation, neurodegenerative diseases, cardiovascular diseases and cancer. To limit these negative effects, exogenous molecules capable of eliminating or neutralizing ROS are an important tool to counteract the onset and control the onset of numerous pathological conditions.

Antioxidant molecules of plant origin are secondary metabolites of plants wherein they perform the function of natural defense against potentially harmful microbes or external stress factors. These molecules typically have a common basic phenolic group, which may be associated with one or more hydroxyl groups. This structure is common to a wide variety of molecules, which can be simple phenolic molecules or complex polycyclic structures having different hydroxyl and phenolic groups.

Previous studies have shown that grapes, the fruit of vine (*Vitis vinifera L. subsp. vinifera*), is one of the fruits with the highest content of phenolic antioxidant molecules, such as phenolic acids, flavonoids, stilbenes, anthocyanins and tannins. Several studies have confirmed that, during the winemaking process, these phenolic substances present in the grapes remain mostly in the pomace and only a small part pass into the wine. Thanks to this high content of phenolic molecules, pomace represents a precious eco-sustainable and low-cost source of natural antioxidants, which can be separated in the form of pure compounds or phytocomplexes.

The same happens for waste from the processing of other plant species such as, for instance, artichoke, tomato and olive processing waste. These wastes are still rich in both hydrophilic and lipophilic biologically active substances which together constitute the phytocomplexes beneficial for humans and animals. Waste generally has a certain water content and contains all parts of plants, such as roots, flowers, leaves, stems, fruits and their parts to varying degrees depending on the type of plant used and the industrial product obtained. These wastes contain fibrous parts such as peels, seeds, cores and stems, which are rich in bioactive substances and antioxidants.

The recovery of phenolic molecules from waste or agro-industrial processing by-products, such as pomace but not only, can contribute to the valorisation of the waste material, and at the same time allows the use of a low-cost matrix to manufacture food products, cosmetics and pharmaceuticals. However, the extraction method plays an important role in the yield and cost of the extract: it should make it possible to obtain the maximum yield and quality of active substances, using non-toxic solvents and inexpensive procedures, in order to guarantee efficacy and commercial suitability of the final product.

Perra M. et al in "Extraction of the antioxidant phytocomplex from winemaking by-products and sustainable loading in phospholipidic vesicles specifically tailored for skin protection" Biomedicine & Pharmacotherapy Available online 29 July 2021 https://doi.org/10.1016/j.biopha.2021.111959 teaches that in order to obtain an antioxidant phytocomplex from pomace and then convey it in a suitable carrier, it is necessary to use three separate and consecutive preparatory steps: the extraction of the bioactive substances, the elimination of the solvent to obtain the dry extract and the subsequent loading of the previously dried extract into phospholipidic vesicles.

MANCA M. et al. in "From waste to health: sustainable exploitation of grape pomace seed extract to manufacture antioxidant, regenerative and prebiotic nanovesicles within circular economy", SCIENTIFIC REPORTS, vol. 10, no. 1, 1 January 2020 (2020-01-01), XP093023890, DOI: 10.1038/s41598-020-71191-8 teaches that to obtain an antioxidant phytocomplex from pomace and then convey it in a suitable carrier it is necessary to freeze-dry the biomasses, grind them 5 minutes to obtain a homogeneous powder, put them to macerate in ethanol, keep them under constant stirring for 48 hours, filter and centrifuge to separate the residual biomasses from the ethanolic solution, eliminate the solvent from the extractive solution first by evaporation then by freeze-drying, mix the dry extract obtained with the phospholipid, surfactant and hyaluronic acid, hydrate all these components with the aqueous mixture and sonicate the dispersions obtained.

MANCONI M. et al. in "Nano-incorporation of bioactive compounds from red grape pomace: In vitro and ex-vivo evaluation of antioxidant activity, INTERNATIONAL JOURNAL OF PHARMACEUTICS, ELSEVIER, NL, vol. 523, no. 1, 20 Pomaceh 2017 (2017-03-20), pages 159-166, DOI: 10.1016/J.IJPHARM.2017.03.037 teaches that to obtain an antioxidant phytocomplex from pomace and then convey it in a suitable carrier it is necessary to macerate the pomace in a mixture of ethanol and propylene glycol, keep them under constant stirring for 48 hours, then centrifuge to separate the pomace from the extractive solution, re-suspend the residual pomace in olive oil, leave them to macerate for another 48 hours, separate again the residual pomace from the olive oil by centrifugation, disperse the two extractive solutions (ethanol with propylene glycol and olive oil) with the phospholipid, hydrate all these components with the aqueous mixture and sonicate the obtained dispersions.

Another method known and reported in the literature involves three phases, a first extraction in a hydrophilic vehicle, a second in a lipophilic vehicle and then the final incorporation into the vesicles. In this case, the pomace is first macerated in ethanol and propylene glycol and subsequently in extra virgin olive oil, in order to extract both the hydrophilic and lipophilic compounds. The two extraction solutions are then used to prepare the phospholipidic vesicles, in order to combine in a single system the beneficial properties of both hydrophilic and lipophilic antioxidant phenolic molecules and those of fatty acids.

From the prior art it is known that phospholipids dispersed in water spontaneously form bilayers which close on themselves to form uni- or multi-lamellar phospholipidic vesicles, commonly called liposomes, which are capable of carrying both hydrophilic and lipophilic drugs. According to what is reported in the literature, liposomes are mainly prepared with quite complex processes which use several steps and which require the use of organic solvents. Furthermore, these systems are used to convey one or more active substances, with variable molecular weight, and in generally limited concentration. Very often liposomes are modified, complexed or covered with polysaccharide polymers such as, for instance, chitosan, alginate, xanthan gum and cellulose derivatives, to protect the phospholipids themselves and the liposomal structure from the external environment and to modify their surface properties.

The patent document EP-818225 reports a method for obtaining a lipophilic extractive liquid rich in lycopene starting from dry peels of *Lycopersicum esculentum* using normal-hexane and soy lecithin (0.01 %). The extraction is done with normal-hexane and a small percentage of lecithin.

The patent document EP-3518949 A1 describes an extraction method for lipophilic (or water insoluble) substances starting from different natural sources using a "unique one pot" process. In this case the vegetable biomasses are dispersed in a mixture of paraffin, tween 80, PEG200, phospholipid and alcohol. The extractive mixture is homogenized for 30 minutes at 40°C and subsequently the solid component is removed by centrifugation. The extraction processes described in both documents allow to obtain predominantly lipophilic active substances, in fact, the extraction media are lipidic and can contain small quantities of surfactants or solvents miscible with water such as ethanol.

Furthermore, according to what is reported in these patent documents, the extracts thus obtained are used in conventional pharmaceutical, cosmetic and nutraceutical formulations, which certainly facilitate the administration of the active substances but do not increase their bioavailability and effectiveness and are not able to avoid their degradation if they are unstable, as happens for many secondary metabolites of plants.

The present invention proposes the preparation of a system, which is able to solve all the problems posed by the prior art with the setting up of a one-shot extraction and delivery process starting from a vegetable matrix, mainly a waste of agro-industrial processing (e.g. pomace). The proposed process allows:

- to extract from the biomasses with a single step a good part of the components of the phytocomplex, both hydrophilic molecules and lipophilic molecules,

- to extract antioxidant molecules and phytocomplexes,

- to obtain phytocomplexes containing high concentrations of antioxidant substances,

- to simultaneously load these substances in the aqueous compartment or in the lipid compartment of the phospholipidic vesicles, to directly convey these bioactive substances from the plant matrix to the vesicular structure where they are solubilized in the aqueous core or intercalated between the lipophilic tails of the phospholipids inside the bilayer lamellar or adsorbed on the surface of the vesicular membrane.

In this way specific interactions are created between vesicles-extract which make the extractive vesicles peculiar and different from the phospholipidic vesicles which are instead obtained by adding an extract obtained with an extraction process which follows the teachings of the prior art. In the latter case the extract will contain only a part of the components of the phytocomplex, for instance in the case of Perra et al and Manca et al. the more hydrophilic ones, so that most of the components of the phytocomplex will be solubilized or will interact with the polar portion of the phospholipids, therefore the internal structure of the vesicles will be different because the most lipophilic component of the phytocomplex is missing. Several previous studies have shown that lipophilic molecules, even of natural origin such as terpenes, intercalate very well in the lipid bilayer of lamellar vesicles and improve their delivery performances. With the method proposed in the present invention, thanks to the use of extractive phospholipidic vesicles, a phytocomplex richer in active substances is obtained, which can be both lipophilic and hydrophilic in nature and which are distributed in the internal aqueous phase of the vesicles or in the lipidic bilayer of the vesicular membrane. In fact, the phospholipidic vesicles used as an extractive system that is formed extemporaneously are the key point of the invention. They are formed by a double layer of phospholipids, which is able to incorporate all the lipophilic molecules of the phytocomplex inside it, while the hydrophilic ones can be solubilized in the aqueous compartment enclosed by the membrane. Their extemporaneous formation in the extractive mixture allows to improve the extractive yield in qualitative and quantitative terms. Furthermore, the extractive vesicles obtained can increase the bioavailability of the carrier substances because they convey them across biological membranes.

Up to now, in the literature, phospholipids have been used for the delivery of phytocomplexes (also deriving from grapes and obtained with different methods) thus obtaining liposomes, ethosoms, glycerosomes, PEV (Penetration enhancercontaining vesicles), or phytosomes. These systems have proven to be excellent carriers capable of improving the bioavailability of phytocompounds, which are generally poorly soluble in biological fluids and do not easily cross biological membranes. In the present invention, the phospholipidic vesicles have been used as systems for improving the qualitative-quantitative yield of the extraction process, as well as as loading and convey systems (carriers) for improving the bioavailability of the carrier substances.

Therefore, the invention aims to solve the technical problems associated with the poor extraction efficiency of the phytocomplex starting from vegetable biomass or agro-industrial processing waste such as pomace and at the same time allows to increase the value of a substrate, which otherwise would require resources, materials and economics for disposal.

Unless specifically excluded in the detailed description that follows, what is described in this chapter is to be considered as an integral part of the detailed description of the invention.

Summary of the Invention

The problems of the prior art are solved with a process according to the invention wherein in a single step the bioactive substances are extracted from a vegetable matrix (e.g. pomace or artichoke) and simultaneously incorporated into the phospholipidic vesicles obtaining a phytocomplex rich in bioactive molecules, especially antioxidants and already conveyed in suitable nanocarriers (extractive vesicles).

The process is carried out by macerating the vegetable biomass in an aqueous solution containing water, water-miscible food-grade solvents and one or more phospholipids.

An object of the present invention is therefore to provide a process which allows the simultaneous extraction of the phytocomplex contained in vegetable biomasses such as agro-industrial processing waste and their conveyance in phospholipidic vesicles (extractive vesicles) as claimed in claim 1.

Another object of the invention are the compositions comprising the extractive vesicles obtained with the process described in the invention.

A further object of the invention is the use in the pharmaceutical, nutraceutical and cosmetic fields of the extractive vesicles thus obtained and of the corresponding compositions.

These and other objects, advantages and features of the present invention will be better specified in a detailed description of the preferred embodiments, which follows. Furthermore, the claims describe preferred variants of the invention, forming an integral part of the present description.

Brief description of the Figures

The purposes and advantages of the present invention will become clear from the detailed description which follows and reports an example of preparation of the same (and of its variants) and from the annexed drawings, provided for purely explanatory and non-limiting purposes, wherein:

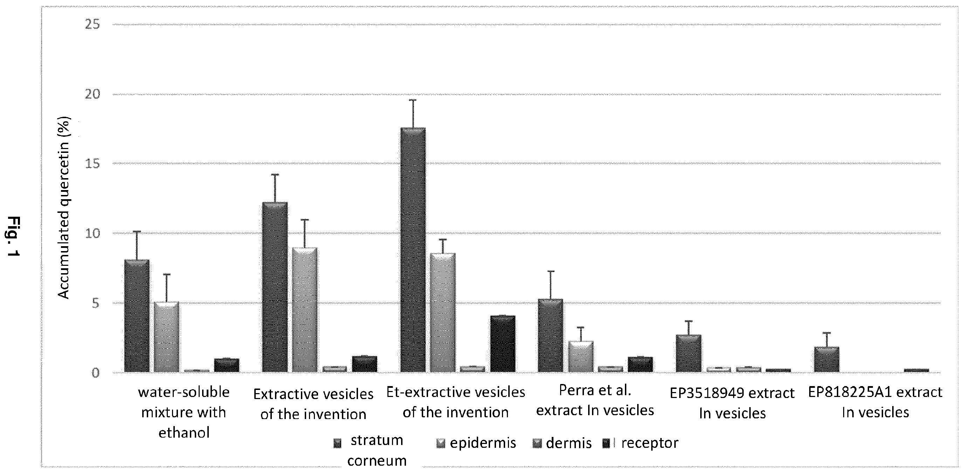

Fig. 1. Graph of the concentration (%) of quercetin accumulated in the stratum corneum, epidermis and dermis or permeated into the receptor compartment after 8 hours of application of the Extractive vesicles according to the invention, prepared with (Et-extractive vesicles) or without (Extractive vesicles) ethanol or the corresponding extractive control solution (comparison) prepared without phospholipids and with ethanol, of the phospholipidic vesicles carrying the extract obtained with the method of Perra et al. (prior art), phospholipidic vesicles carrying the extract obtained with the method of the patent EP3518949 A1 and phospholipidic vesicles carrying the extract obtained with that of patent EP-818225 A1. Mean values (bars) $\pm$ standard deviations (error bars) were reported (n=6);

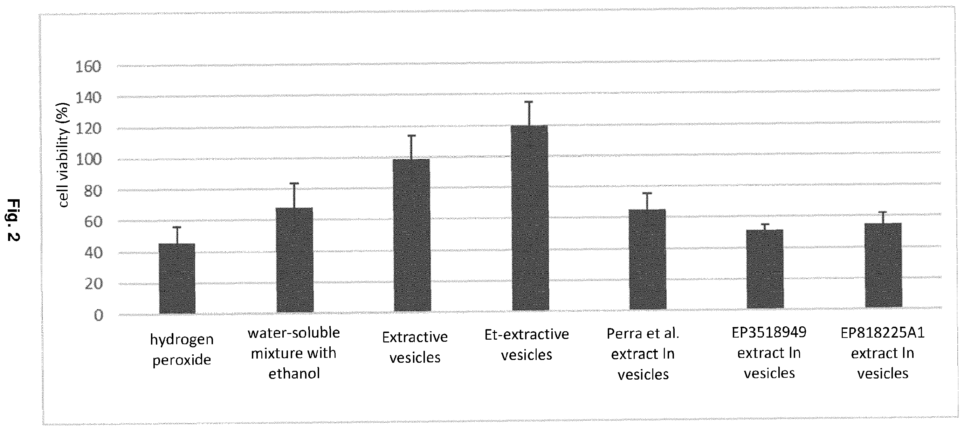

Fig. 2. Graph of the viability of the epidermal cells stressed with hydrogen peroxide and treated with the Extractive vesicles according to the invention, prepared with (Et-Extractive vesicles) or without (Extractive vesicles) ethanol or with the corresponding extractive control solution (comparison) prepared without phospholipids and with ethanol, with the phospholipidic vesicles carrying the extract obtained with the method of Perra et al. (prior art), with the phospholipidic vesicles carrying the extract obtained with the method of the patent EP3518949 A1 and with the phospholipidic vesicles carrying the extract obtained with the method of the patent EP-818225 A1. Mean values (bars) $\pm$ standard deviations (error bars) were reported (n=6).

Detailed description of the invention

In the context of the present invention, the term "one-shot process" means a single process, which simultaneously involves the extraction of the phytocomplex and its delivery in the phospholipidic vesicles.

In the context of the present invention, the term "nanocarrier" refers to particle systems of nanometric dimensions capable of loading and conveying hydrophilic or lipophilic molecules.

In the context of the present invention, the term "phospholipidic vesicles" refers to uni- or multi-compartmental lamellar vesicles of nanometric dimensions, formed by one or more double layers of phospholipids closed on themselves, concentric and separated by aqueous layers. These vesicles, thanks to their dual nature (hydrophilic and lipophilic) are able to simultaneously convey both hydrophilic and lipophilic molecules.

In the context of the present invention, the terms "extractive vesicles" and "extractive phospholipidic vesicles" mean lamellar phospholipidic vesicles which are formed directly in the extractive mixture and simultaneously extract and convey within them the lipophilic substances in the bilayer and the hydrophilic ones in the aqueous compartment. Said vesicles are those obtained with the process of the present invention.

In the context of the present invention, the term "agro-industrial processing waste" refers to the residual vegetable biomass, which is produced during the processing and transformation of vegetable material to obtain commercial products such as wine, olive oil, saffron, tomato puree. Pomace are the preferred biomasses in the application of the present invention, therefore the terms "waste/s from agro-industrial processing" and "biomass/es" are to be considered synonymous.

In the context of the present invention, the term "pomace" refers to the by-products of the winemaking process, which are obtained after racking off or the exhausted pomace deriving from distillation processes. Pomace contains seeds, skins and grape seeds.

In the context of the present invention, the term "phytocomplex" means the set of chemical components contained in a vegetable biomass, part of which have generally different and complementary or synergistic biological or pharmacological activity and part of which have no biological activity (such as fiber or cellulose) but can help enhance the effectiveness of the blend. Sometimes the synergistic whole of the phytocomplex can be biologically more effective than the single active substances.

In the context of the present invention, the term "antioxidant activity" refers to the ability of natural polyphenolic substances to reduce and counteract, slow down or neutralize the formation of oxygen radicals which are formed following oxidation reactions. Their antioxidant activity is measured as a function of their ability to react and decolorize specific reagents which decolorize when reduced in the presence of an antioxidant molecule (Measurement of antioxidant activity)

In the context of the present invention, the term "oxidative stress" means the condition wherein there is an accumulation of radical species generated by hydrogen peroxide and which are harmful to cells. Oxidative stress is measured as a function of the cellular survival from the damage caused to cells or their molecules (bibliographic reference: Hydrogen Peroxide Induced Oxidative Stress Damage and Antioxidant Enzyme Response in Caco-2 Human Colon Cells).

In the context of the present invention, the term "about" means a numerical value equal to the reported value ± its standard deviation.

In the process of the present invention, the biomasses deriving from or constituting the agro-industrial processing waste, in particular the waste (biomass) deriving from the viticulture or distillery processes, preferably pomace, are:

a) dispersed in an aqueous solution, preferably containing water and food-grade water-miscible solvents such as for instance glycerol and/or propylene glycol and/or ethanol, and one or more phospholipids, preferably soy lecithin;

b) left to hydrate at room temperature from 0.5 to 2 hours, and then kept under stirring for 1 to 4 hours at a controlled temperature between 30 and 50°C, to favor the extraction of the phytocomplex and the formation of vesicles.

c) centrifuged for about 30-60 minutes at 500-10,000 rpm to separate the exhausted biomass from the dispersion containing the extractive vesicles.

d) Possibly purified by dialysis.

Preferably, the biomasses to be treated are hydrated solids and have a water content which can vary from about 2 to about 20% by weight.

The extractive solution and vesicle moisturizer is a mixture containing at least 50% water (preferably 50-98% v/v), food grade glycols, such as propylene glycol and/or polyethylene glycol and/or glycerol (1-30 % v/v), ethanol (0-20% v/v). These components constitute 100% of the extractive solution, which may possibly include other components.

The one or more phospholipids are added in quantity (6-9% w/v) with respect to the extractive solution.

The extractive mixture and one or more phospholipids are added to the biomasses in quantities of 2-30% w/v with respect to the biomasses so that there is a dispersion of the biomasses in the combination of extractive mixture and phospholipids.

Outside the indicated ranges there is no adequate extraction and above all the phospholipidic vesicles may not form, the phospholipids may aggregate in other non-lamellar and non-vesicular forms or the vesicles may form but have non-optimal characteristics for the delivery of bioactive substances, especially too large or unstable.

In summary, the extraction vesicle production process comprises the basic steps of:

i. Preparing a water-soluble mixture containing water in an amount greater than 50%, preferably 50-98%v/v, one or more food grade glycols, such as for instance propylene glycol and/or polyethylene glycol and/or glycerol in an amount of 1-30 % v/v, ethanol in amount 0-20% v/v;

ii. Preparing a hydrated biomass starting from vegetable material processing waste whose water content is about 2-20% w/v with respect to the solid material of the biomass;

iii. Adding to the hydrated biomass an extractive mixture in a quantity of 2-30% w/v with respect to the biomass so as to obtain a mixture being treated, said extractive mixture being constituted by 100% of the water-soluble mixture of stage i.) to which 6-9% w/v of one or more phospholipids with respect to said water-soluble mixture is added; outside the indicated ranges there is no adequate extraction and above all the phospholipidic vesicles may not form, the phospholipids may aggregate in other non-lamellar and non-vesicular forms or the vesicles may form but have non-optimal characteristics for the delivery of bioactive substances, especially too large or unstable.

iv. Leaving the mixture being treated in step iv.) to hydrate at room temperature for about 0.5-2 hours, possibly under stirring;

v. Then stirring the mixture of the previous stage for 1-4 hours at 30-50°C, to favor the extraction of the phytocomplex and the formation of vesicles;

vi. Separating the exhausted biomasses from the dispersion containing the extractive vesicles, for instance, by centrifugation or other similar method and recovering the extractive vesicles;

vii. Possibly purifying the vesicles with known methods, for instance by dialysis.

The preferred water-miscible solvents are selected from propylene glycol and/or glycerol, ethanol (0-20% v/v), the phospholipids are preferably soy lecithin.

The biomass (2-30% w/v) comprises one or more of the following components: seeds, leaves, fruits, flowers, branches, stems, roots, bark present in the vegetable waste to be treated.

According to an embodiment of the invention, by dispersing together at 40°C pomace (20, 100 and 200 mg/ml) and soy lecithin (60-90 mg/ml) in a mixture of water, glycerol and propylene glycol and (optionally) ethanol extractive vesicles carrying a phytocomplex rich in antioxidant substances were obtained and tested experimentally. Lecithin and pomace were used in different concentrations in order to find the most suitable formulation to extract the greatest quantity of active substances. Average diameter, zeta potential and composition of the phytocomplex conveyed in the obtained formulations were measured.

The composition of the three formulations of extractive vesicles which allowed to extract the phytocomplex qualitatively and quantitatively richest in bioactive substances is shown in table 1. Uni- or multi-lamellar vesicles were obtained, sometimes multi-compartmental, with average dimensions ranging from 50 to 500 nm; preferably from 80 to 300 nm; more preferably, 100 to 250 nm.

In the process according to the invention one or more food grade phospholipids can be used such as for instance those selected from phosphatidylcholine, phosphatidylethanolamine, phosphatidylglycerol, phosphatidylserine, phosphatidylinositol, their derivatives, mixtures thereof also containing other lipids such as cholesterol, phytosterol, oleic acid, linoleic acid, squalene and carotenes. The at least one phospholipid is preferably selected from the group consisting of one or more natural or synthetic phospholipids, pure or in mixtures, such as, for instance, soy or egg lecithin, phosphatidylcholine in various degrees of purity, hydrogenated or not hydrogenated, and all of its derivatives, phosphatidyl-ethanolamine, phosphatidyl-serine, phosphatidyl-glycerol, phosphatidyl-inositol, dimyristoyl-phosphatidyl-choline, dipalmitoyl-phosphatidylcholine, distearoyl-phosphatidyl-choline, palmitoyl-stearoyl-phosphatidyl-choline, sphingomyelin, mixtures of phospholipids, hydrogenated and non-hydrogenated, derived from soy, and/or mixtures of the above phospholipids, for instance, the products known under the trade name of Phospholipon 90G (P90G) or Lipoid S75 (S75) (two mixtures of phospholipids, mainly containing soy phosphatidylcholine and distributed in Italy by AVG S.r.l., Garbagnate Milanese on behalf of Lipoid GmbH, Ludwigshafen, Germany); preferably, soy phosphatidylcholine, hydrogenated or not, its derivatives described above, mixtures thereof, and/or the commercial products P90G or S75 or unpurified soy lecithin commonly sold as a food supplement. The most abundant phospholipid is phosphatidylcholine, also known as lecithin, and it is the preferred phospholipid for the preparation of these vesicles.

According to the invention, extractive vesicles formed by lamellar membranes closed to form vesicles with dimensions ranging from 80 to 400 nm are obtained. The membrane can be single (mono-lamellar) or multiple, concentric membranes (multi-lamellar) can form and the vesicles are homogeneously dispersed in an aqueous medium. The vesicles encapsulate in their central aqueous compartment or between the lamellae water-soluble bioactive substances and in the lamellar lipid bilayer, lipophilic bioactive substances. The bioactive substances extracted and conveyed can be: anthocyanins, flavonoids, hydroxybenzoic acids and in addition they can include other phytocomplexes, water-soluble vitamins, fat-soluble vitamins, macrominerals, trace elements, phytochemicals, amino acids, fatty acids, peptides, vegetable extracts and other bioactive components of various nature, characteristic of the biomass being treated. Therefore, the vesicles of the invention convey together several bioactive substances of different chemical nature forming an ideal delivery system for the administration of the active substances in pharmaceutical, cosmetic or nutraceutical products.

The extractive vesicles according to the invention deliver a phytocomplex which contains high concentrations of polyphenols, especially anthocyanins and flavonoids which have a high antioxidant activity.

After adding a suitable cryoprotectant such as glucose, mannose, dextrose, lactose, dextrin, the dispersion of the phospholipidic vesicles of the present invention can advantageously be freeze-dried, using standard methods and equipment commonly employed in the art, to obtain freeze-dried solid complexes. In this form, the phospholipidic vesicles of the present invention are particularly stable and can be stored, for instance under vacuum, for a long time and can be reformed extemporaneously by adding water, before use, or directly inside the body when they are in contact with biological fluids.

They can therefore be used in freeze-dried or rehydrated form in the preparation of nutraceutical and/or pharmaceutical and/or cosmetic products intended for oral or topical administration, for the treatment of all conditions or affections which need or benefit from the administration of antioxidants.

Consequently, these freeze-dried solid complexes are also an object of the present invention, as well as their dispersion extemporaneously reconstituted before use.

The process of the invention allows the phytocomplex contained in pomace to be extracted and conveyed in a single step through the addition of phospholipids (e.g. soy lecithin) to the water-based extractive solution. Phospholipids have the dual function of increasing extraction efficiency and allowing it to be conveyed inside the vesicles.

Thanks to the addition of phospholipids it is possible to avoid the use of apolar and toxic organic solvents, obtaining however a phytocomplex rich in active substances which is already incorporated in phospholipidic vesicles. The latter are able to convey the active substances within the body, facilitate their passage through biological membranes, increase their bioavailability, their accumulation in target tissues and therefore their effectiveness. The process of extraction and delivery of the phytocomplex is carried out in a single step, obtaining a concentrated, green and ready-to-use product.

The extract conveyed in the phospholipidic vesicles is obtained:

i. through a single production process that simultaneously includes extraction and delivery;

ii. adding to the aqueous extractive solution (containing water and water-miscible food-grade solvents) the phospholipids (substances of natural and highly biocompatible origin) which act as promoters and allow a better extraction efficiency and at the same time the formation of the ready-to-use delivery system;

iii. without using energy-intensive and dissipative processes divided into different sequential steps that are usually used for the extraction before and the delivery after, such as: extraction at high temperatures, removal of the solvent, mixing with phospholipids, hydration and sometimes extrusion.

Extraction is a low-cost ecological extraction of bioactive compounds from grape pomace or other vegetable matrices obtained from agro-industrial processes and occurs simultaneously with their incorporation into nanometric phospholipidic vesicles. The overall results demonstrate the potential of extractive vesicles carrying antioxidant phytocomplexes for the development of an antioxidant product, which can be used for pharmaceutical and/or cosmetic and/or nutraceutical applications.

From a careful bibliographic research, it seems that phospholipidic vesicles have never been used to increase the extractive yield of phytocomplexes. Their application in this process therefore provides the double advantage of:

- facilitating the extraction obtaining a richer and more concentrated phytocomplex;

- favoring the absorption of the components of the phytocomplex in the intestine;

- promoting the penetration of the components of the phytocomplex through the layers of the skin or mucous membranes and their local accumulation.

The innovation of the process of the invention lies in the addition of phospholipids (such as for instance soy lecithin) to the aqueous or hydroalcoholic extractive solution which allows to increase the extraction efficiency of the phytocomplex, without using toxic solvents and allows to obtain as a result a phytocomplex already incorporated in phospholipidic vesicles. The latter are able to increase its accumulation, its bioavailability and therefore its effectiveness on the skin.

It is known that liposomes are generally particularly unstable in the gastrointestinal environment, because phospholipids are easily hydrolysed, consequently the vesicles break, thus releasing the substances they carry. To avoid this drawback, the vesicles of the invention can be advantageously complexed or protected with specific polymers which make them gastro-resistant. An exemplary and non-exhaustive list of products that can be used is: chitosan, cyclodextrin, heparin, chondroitin, arabic gum, xanthan gum, tragacanth gum, alginate, gelatin, zein, cellulose, starch, carboxymethylcellulose.

Therefore, a further specific object of the present invention is the use of vesicles for the preparation of cosmetic and/or nutraceutical products for use as antioxidant and/or pharmaceutical supplements intended for oral or topical administration, for use in prevention and treatment of diseases that benefit from the administration of antioxidant compounds. These substances are able to protect tissues, cells and their fundamental constituents, especially lipids and DNA, from damage caused by oxidative stress. In this way, first of all, premature aging, irritation, cellular dysbiosis and the onset of chronic inflammatory conditions are prevented, which in the long term facilitate the onset of chronic diseases, such as cardiovascular, metabolic, neurodegenerative and tumoral diseases. Antioxidants also play a crucial role in the skin, which is the organ of the human body that interacts with the external environment and is subjected to chemical, physical and mechanical aggressions, which generate the accumulation of highly reactive free radicals (oxidative stress). The phenolic compounds contained in the present invention are useful in neutralizing these species and preventing damage caused by oxidative stress and therefore are able to prevent, counteract or control both physiological and pathological skin aging and the onset of dysfunctions or chronic pathologies. Aging, of any type, can lead to a decrease in the structural integrity of the skin and loss of physiological function. Aged skin becomes dry, atrophic, dysfunctional and more susceptible to disease.

The results of studies conducted to measure the ability of Extractive vesicles to protect skin cells from damage caused by oxidative stress are shown in Figure 2. When cells are treated simultaneously with hydrogen peroxide and Extractive vesicles, cell viability is ~ 100%, a result certainly superior to the corresponding formulations of the prior art, moreover, the value rises further up to ~120% when the Et-extractive vesicles are used. So the extractive vesicles completely cancel the effect of the oxidative damage, avoid cell death and even the Et-extractive vesicles promote cell proliferation. This does not occur when using the extractive solution without phospholipids and containing ethanol and the other vesicles obtained by means of the prior art.

The data presented here allow to state that the extractive vesicles produced with the method of the invention are not only different, but also better performing than the phospholipidic vesicles of the prior art, thus confirming the fact that the process parameters and steps described here result in another product (the demonstration that there are evident differences in the properties of the products obtained is evident, for instance, from table 5).

The present invention also relates to extractive vesicles or compositions comprising them as described above, for use in the treatment of skin disorders. Treatments can be both medical and cosmetic. Among the medical treatments we can list the treatment of cicatricial alterations and/or preventive and curative treatment of the keloid; the treatment of atopic and seborrheic dermatitis; the symptomatic treatment of some autoimmune diseases such as psoriasis. Examples of cosmetic treatments based on the use of vesicles according to the present invention are treatments with an anti-aging function, with an antioxidant function, with a draining activity, with a moisturizing and nourishing function or with a post-peeling soothing function.

The extractive vesicles of the present invention can be used in combination with allantoin or ellagic acid and formulated together in a gel of hyaluronic acid and chondroitin to prepare a device for the treatment of radiodermatitis. Or, they can be dispersed in a mucilage of arabic gum and tragacanth gum which protect them from the harmful action of the gastrointestinal environment and mannose can be added to the dispersion to avoid breakage during freezing. The dispersions obtained can be freeze-dried and the lyophilisate can be inserted into gastro-resistant capsules to obtain a food supplement of antioxidants with high intestinal absorption.

The extractive vesicles carrying the active phytocomplexes after dispersion in a structured vehicle of hyaluronic acid constitute a stable semi-finished product for obtaining various cosmetic formulations such as face serums, eye contour serums, creams and face masks.

For instance, the composition of an antioxidant face serum could be:

Water 84.9%
vesicular dispersion (extractive vesicles) 10%
sodium hyaluronate 0.8%
Glycerol 3%
Benzyl alcohol 0.9%
Ethyl-hexyl-glycerin 0.1%
Perfume 0.1%

Again by way of example, the composition of a light anti-aging face cream could be:

Water 69.9%
vesicular dispersion (extractive vesicles) 10%
Sunflower Oil 5%
Tocopherol acetate 1%
Argan oil 5%
Sorbitan oleate 4%
sodium hyaluronate 1%
Glycerol 3%
Benzyl alcohol 0.9%
Ethyl-hexyl-glycerin 0.1%
Perfume 0.1%

The vesicles can be formulated for oral, sublingual, buccal administration or for topical application to the skin or mucous membranes.

The vesicles can be formulated for oral administration with suitable excipients followed by the incorporation of the dry powder into a capsule, tablet or granule. Or they can be formulated for sublingual administration in solid granulates obtained with excipients and binders in powder form or for buccal administration incorporated in tablets, lollipops, chewing gums, gels and films.

The incorporation of the vesicles into compositions for the uses indicated above may comprise one or more of the following components:

- suitable emulsifiers such as polysorbates, monoglycerides, diglycerides, triglycerides, ethylene oxide/propylene oxide copolymers, sorbitan ethylene oxide/propylene oxide copolymers, alkylaryls, polyether alcohol polymers, bile salts, alcohols and other surfactants known to the art;

- preservatives such as tocopherols, ascorbyl palmitate, sorbates, parabens, optiphen, timersal, benzoic acid, benzalkonium chloride, polyquaternium-1, ethyl lauroyl arginate and rosemary oleoresin. Preferred preservatives are:

potassium sorbate, phenoxyethanol, ethylhexylglycerin, propanediol, phenethyl alcohol, undecyl alcohol, benzyl alcohol, ethylhexylglycerin, benzyl alcohol, dehydroacetic acid;

- sweeteners for intraoral and oral routes of administration to improve consumer acceptability. The sweeteners used can be natural sweeteners or artificial sweeteners. Natural sweeteners include xylitol, stevia, sucrose, fructose, fructo-oligosaccharides, glucose, glucose syrup, inverted sugar, maltodextrin, magnasweet, erythrol, sorbitol, maltitol, lactitol, mannitol, and isomaltol. Examples of artificial sweeteners include sucralose, aspartame, acesulfam K, neohesperidin salts, dihydrochalcone, thaumatin, saccharin, and saccharin. The preferred sweeteners are natural sweeteners such as xylitol, erythritol, stevia and magnasweet. Typically the sweetener content will be between 0.05 and 2.5% w/w;

- flavors for intraoral and oral routes of administration to improve consumer acceptability. The flavors used can be natural sweeteners or artificial sweeteners. Examples of flavoring agents useful in the compositions include fruit concentrates (e.g., pineapple or citrus) and aqueous or nonaqueous flavor concentrates such as aromatic oils. Typically the flavoring content will be from 0.1 to 2% w/w;

- polymers useful for the preparation of nanoparticles as carriers of nutraceuticals are natural hydrophilic polymers and synthetic hydrophobic polymers. Examples of natural hydrophilic polymers as nutraceutical carriers include proteins (gelatin, albumin, lecithin, legumin and vicillin) and polysaccharides (alginate, dextran, chitosan, agarose and pullulan). Examples of synthetic hydrophobic polymers as nutraceutical carriers include PLGA (poly-d,1-lactide-co-glycolide), PLA (polylactic acid), PCL (polycaprolactone), PAC (polyalkylcyanoacrylates), polyisobutyl cyanoacrylates, polybutylcyanoacrylates and polymethyl-(methcyanoacrylates);

- additional polymers having the function of interacting with phospholipids making the formulation gastro-resistant and ensuring greater stability, in particular during transit in the acidic environment of the stomach. Said additional polymer is preferably selected from one or more known polymers, such as, for instance, cellulose derivatives, alginates, gums, copolymers of methacrylic acid and its methyl ester (known under the commercial name of Eudragit®, Evonik Industries, Essen, Germany), and/or mixtures thereof; preferably, hydroxypropyl methylcellulose and Eudragit®;

- emulsifiers suitable for stabilizing the final emulsion, which are selected, but are not limited to: cetearyl oleate, sorbitan oleate, glyceryl stearate, triglyceryl diisostearate, polyglyceryl transesterified with sorbitol, polyglyceryl oleate, cetyl alcohol, methylglucose sequistearate, cetearyl oleate.

According to the invention the vesicles can be formulated in specific formulations which facilitate the application and lengthen the residence time at the application site itself of the pharmaceutical, nutraceutical and cosmetic substances. In particular, formulations for topical use can be made viscous and modified to increase bioadhesion on the skin and facilitate the passage of vesicles from the formulation to the skin.

The stratum corneum is the main barrier to percutaneous absorption of topically applied compounds. It has been demonstrated that the extractive vesicles of the invention are particularly effective in conveying the phytocomplex contained therein through the skin, facilitating the crossing of the stratum corneum and allowing greater accumulation in the deeper layers of the skin, where the active substances can carry out their protective and beneficial action. In fact, these vesicles are able both to fluidify the stratum corneum and to penetrate intact through its lamellae, allowing the delivery of bioactive substances, both lipophilic and hydrophilic.

For topical administration, the Extractive vesicles can be formulated in the form of serums, fluid lotions, foams, gels, soaps, scrubs, milks and creams prepared according to standard procedures and suitable for application to the skin of a mammal, including humans including newborns, infants and the elderly. The formulation in gel or serum is particularly suitable. The serum can be formulated by simply adding the dispersion of the extractive vesicles to a 1% hyaluronic acid gel previously enriched with chelators and preservatives. The scrub can be obtained by adding the dispersion of the extractive vesicles to a lipidic emulsifying hydrophilic gel, containing coarsely ground grape seeds, which help to eliminate the superficial layer of the dead skin. The hydrophilic lipid-emulsifying gel is an intermediate system between gel and cream because, despite being predominantly hydrophilic, thanks to its surfactant structure, it is capable of incorporating and permanently dispersing even small quantities of fat molecules within itself. Thanks to its high water content associated with a small proportion of lipids, it is particularly pleasant on the skin and not greasy. Its lipidic portion forms a thin film on the skin with a moisturizing action while the hydrophilic component evaporates quickly and gives a refreshing effect.

The extractive vesicle compositions prepared according to the invention include one or more cosmetically and pharmaceutically acceptable adjuvants and carriers, such as one or more oil components, aqueous components, emulsifiers, hydrophilic components, preservatives, active agents, propellants, buffers, or salts, viscosity modifiers or other agents such as fragrances.

The formulation may also contain one or more of the following components: antibiotic agents, antimicrobial agents, antibacterial agents, antifungal agents, antiinflammatory agents; preservatives and antioxidants. The following can be added: pH regulating agents in the quantities necessary to obtain the desired pH, generally a physiological pH between 5 and 7, preferably between 6 and 7, where lactic acid is preferred; viscosity modifiers, such as xanthan gum.

The oily components which can be used for the formulation can be selected from the following groups: linear or branched hydrocarbons, fatty acids, mineral oils, oils of natural origin, waxes, saturated, mono- or poly-unsaturated aliphatic alcohols; vegetable-based oils such as jojoba oil, sunflower oil, rice oil, (rice bran oil) shea butter, cocoa butter, olive oil. Particularly preferred are vegetable oils, such as olive oil, jojoba oil, sunflower oil, corn oil, moringa oil, and waxes and butters, such as candelilla wax, beeswax, shea butter, cocoa butter.

The aqueous components also include, but are not limited to, one or more of the following hydrophilic components: humectants, emollients, water-soluble vegetable extracts, water-soluble active agents, and water-soluble viscosifiers, among which preferred are glycerin, allantoin, xanthan gum, stereraric acid, in a percentage ranging from 50% to 65%, therefore from 50 to 65 g out of a total of 100 g of emulsion. The invention will be further described by the following Examples which are to be considered illustrative and not limiting of the scope of the invention.

EXAMPLES

Experimental section and results obtained using pomace as processing waste

**One shot method**

Pomace of Sardinian Bovale (Cantine Argiolas 2021) was dispersed in a mixture containing water, glycerol, propylene glycol and ethanol (Table 1). Soy lecithin was subsequently added to the dispersion and after one hour at room temperature, the dispersion was vigorously stirred by sonication (99 cycles 5 on 2 off, 13 micron probe amplitude), using a Soniprep 150 sonicator (MSE Crowley, London, United Kingdom) to aid extraction and vesicle formation. Then the dispersion was kept under continuous stirring for 3 hours at 40°C.

At the end of the process, the dispersion was centrifuged twice (30 min, 8000 rpm) to separate the exhausted pomace from the aqueous dispersion containing the vesicles carrying the phytocomplex.

**Composition**

After a preformulation study, a mixture containing water (60% v/v) and propylene glycol (10% v/v) in fixed ratio and glycerol (20-30% v/v) and ethanol (0-10% v/v) in variable ratio was selected as extractive solution and vesicle hydrating medium. The initial quantities of pomace (20, 100 and 200 mg/ml) and of soy lecithin (60-90 mg/ml) were varied until obtaining the best formulations because they were more stable and contained a richer and more concentrated phytocomplex. Table 1 shows the composition of the best extractive mixture.

**Table 1.** Composition of the extractive mixture.

| | Pomace mg/ml | Lecithin mg/ml | Propylen-Glycol ml | Water ml | Glycerol ml | Ethanol ml |
|---|---|---|---|---|---|---|
| Extractive mixture | 200 | 60 | 0.1 | 0.6 | 0.2 | 0.1 |

**Characterization of phospholipidic vesicles**

Vesicle formation and morphology were evaluated using a cryogenic transmission electron microscope (Cryo-TEM). A thin film of our dispersion was deposited on a carbon grid, which was subsequently vitrified by immersion in ethane. The images were acquired at 200 kV and at a temperature --173°C, using an Eagle CCD camera (FEI Company).

The mean diameter and the polydispersion index of the obtained vesicles were measured by Photon Correlation Spectroscopy, using a Zetasizer UltraNano (Malvern Instrument, UK). Before the measurement, the samples were suitably diluted (1:100) to improve the signal quality. The zeta potential was also measured using the Zetasizer UltraNano according to the M3-PALS analysis (Phase Analysis Light Scattering), which measures the electrophoretic mobility of the particles inside a thermostated cuvette. Immediately after preparation, the samples were diluted and analyzed immediately (Table 2).

**Table 2.** Mean diameter (MD), polydispersion index (PI), zeta potential (ZP), and incorporation efficiency (EI) of extractive vesicles obtained with ethanol (Etvesicles) and without ethanol (vesicles). Mean values $\pm$ standard deviations (n≥3) were reported.

|  | MD (nm) | PI | ZP (mV) | EI (%) |
|---|---|---|---|---|
| Extractive vesicles | 126±3 | 0.31 | -45±5 | 95±4 |
| Et-Extractive vesicles | 139±4 | 0.30 | -66±4 | 95±3 |

The unincorporated phytocomplex inside the vesicles was removed from the vesicular dispersions by dialysis. Each dispersion (1 ml) was placed in a polycarbonate dialysis tube (Spectra/Por® membranes: 12-14 kDa MW cut-off, with pores 3 nm; Spectrum Laboratories Inc., USA) and dialysed in two liters of distilled water at 25°C for 2 hours, during which the water was replaced every hour. The incorporation efficiency (EI) was calculated as the percentage of phytocomplex conveyed inside the vesicles, with respect to the initial quantity used for the preparation (Table 2). The amount of the phytocomplex before and after dialysis was quantified by measuring the antioxidant activity with the DPPH (1-2,2-diphenyl-1-picrylhydrazyl) test.

**Antioxidant activity of the formulations**

The antioxidant activity was measured as a function of its ability to decolorize DPPH (1-2,2-diphenyl-1-picrylhydrazyl), a very stable nitrogenous radical, characterized by an intense purple-red colour. DPPH discolors when reduced in the presence of an antioxidant molecule.

The DPPH oxidative assay is used throughout the scientific world for the quantification of the radical-scavenging capacity of molecules, expressed as antioxidant capacity. (C. Peng, S. Chen, F. Lin, Z. Lin Detection of antioxidative capacity in plants by scavenging organic free radical DPPH Progress in Biochemistry and Biophysics, 27 (6) (2000), pp. 57-61). The DPPH alcoholic solution is a deep purple color with an absorption peak at 517 nm, which disappears in the presence of a radical scavenger in the reaction system and when the nitrogen cleavage electrons in DPPH are paired. The reactivity rate and the capacity of the radical scavenger depend on the rate and the disappearance peak value of DPPH (M. Chi, C. Zhang, G. Zheng, X. Mei Determination of radical scavenger in Chinese drug by spectrophotometry China Journal of Traditional Chinese Medicine and Pharmacy, 18 (9) (2003), pp. 567-568). Compared to other methods, the DPPH assay has many advantages, such as good stability, excellent sensitivity as well as simplicity and feasibility in the laboratory (O. Ozcelik, J.H. Lee, D.B. Min Effects of light, oxygen and pH on the absorbance of 2,2-diphenyl-1-picrylhydrazyl Journal of Food Science, 68 (2003), pp. 487-490). Specifically, the formulations (40 $\mu$l) were diluted (1:40) with the methanolic solution of DPPH (40 $\mu$g/mL), the diluted samples were left at room temperature and in the dark for 30 minutes, subsequently the absorbance of the solution was measured at 517 nm, using a UV spectrophotometer. The antioxidant power of the extract was calculated according to the following formula:

$$AA\% = [(ABS_{DPPH} - ABS_{Sample}) / ABS_{DPPH}] \times 100.$$

The antioxidant activity of vesicles prepared with ethanol (Et-Extractive vesicles) and without ethanol (Extractive vesicles) was measured and used to make a first evaluation of the extraction efficacy of the systems. As a comparison, extraction solutions having the same composition as the Extractive vesicles but without the addition of phospholipids were used (Table 3).

**Table 3**. Antioxidant activity of the water-soluble mixtures without phospholipids and with ethanol, used as a comparison, of the extractive vesicles prepared with ethanol (Et-vesicles) and without ethanol (vesicles) and of the vesicles carrying the extracts obtained with the method reported by Perra et al. or in the two patents indicated in the table. Mean values ± standard deviations (n=6) were reported.

|  | Antioxidant activity (%) |
| --- | --- |
| Water soluble mixture without phospholipids with ethanol | 63±2 |
| Water soluble mixture with ethanol | 63±3 |
| Extractive vesicles | 78±3 |
| Et-Extractive vesicles | 82±4 |
| Perra et al. vesicles | 53±2 |
| EP-3518949 A1- vesicles | 23±4 |
| EP-818225 A1- vesicles | 55±4 |

The antioxidant activity of water-soluble mixtures both with and without ethanol and without phospholipids is -63%. The antioxidant activity of the extractive vesicles obtained according to the invention is -78% for those not containing ethanol and increases up to -82% for those containing ethanol. The results confirm a better antioxidant activity of the extractive vesicles of the invention compared to the corresponding water-soluble mixtures without phospholipids, which indicates that more antioxidant substances have been extracted and confirms the fact that the one-shot process of the invention allows to obtain vesicles which from the point of view of the antioxidant activity differ from those obtained with the processes of the prior art.

**Quali-quantitative analysis**

The qualitative-quantitative analysis of the extractive vesicles according to the invention and of the corresponding water-soluble mixtures without phospholipids was carried out using an Agilent 1100 HPLC consisting of a G1311A quaternary pump, a G1313A rheodyne injector, a G1316A column thermostat compartment, a G1322A degasser and a DAD UV 6000 detector (Thermo Finnigan, Milan). A Kinetex column (5u, C18, 100 A, Phenomenex, Torrance, CA, USA) was used and a mixture of acetonitrile (A) and water with 0.22 M phosphoric acid (B) as the mobile phase.

The analysis confirms that the extractive vesicles of the invention, in particular those containing ethanol (Et-extractive vesicles) allow to extract a greater quantity of active substances and in higher quantities (Table 4). The main active substances found in the extractive vesicles are anthocyanin derivatives, delphinidin 3 glucoside and derivatives, quercetin, quercetin glucopyranoside, gallic acid and syringic acid (highlighted in gray the formulation with the best quantitative composition).

**Table 4**. Amounts (mg/kg of extract) of the main antioxidant and phenolic components found (225 nm) in the extractive vesicles (with and without ethanol) and in the corresponding water-soluble mixture containing ethanol. Mean values ± standard deviations (n=3) were reported. Nd indicates an unidentified compound. The symbol * indicates a value expressed as an equivalent of delphinidin-3-glucoside at 520 nm; The symbol ** indicates a value expressed as quercetin glucopyranoside equivalent at 360 nm.

| Compounds | Water soluble mixture without phospholipids with ethanol | Extractive vesicles | Et- extractive vesicles |
|---|---|---|---|
| **Anthocyanins *** | **mg/kg** | **mg/kg** | **mg/kg** |
| **Total Anthocyanins *** | **3428.7** | **6948.2** | **9008.4** |
| Delphinidine derivatives | 203.5 | 368.4 | 398.2 |
| Delphinidine derivatives | 701.6 | 705.5 | 971.3 |
| Delphinidin 3 glucoside | 497.6 | 971.2 | 1127.5 |
| Delphinidin 3-acetyl glucoside | 249.7 | 246.6 | 438.9 |
| Delphinidine derivatives | 105.8 | 285.1 | 291.9 |
| Delphinidine derivatives | 189.3 | 265.8 | 296.4 |
| Delphinidine derivatives | 154.9 | 313.5 | 693.0 |
| Delphinidine derivatives | ND | ND | 482.3 |
| Peonidin-3-glucoside | 169.9 | 406.5 | 567.1 |
| Malvidin-3-acetylglucoside | 144.9 | 308.1 | 554.3 |
| Malvidin 3 glucoside | 1011.5 | 3077.5 | 3187.5 |
| **Flavonoids** | **mg/kg** | **mg/kg** | **mg/kg** |
| **Total Flavonoids** | **368.6** | **901.8** | **1058.9** |
| Iso-quercetin | 60.6 | 153.1 | 245.1 |
| Quercetin derivative ** | 124.4 | 355.0 | 317.4 |
| Quercetin Glucopyranoside | 183.6 | 393.7 | 496.4 |
| **Hydroxybenzoic acids** | **mg/kg** | **mg/kg** | **mg/kg** |
| **Total hydroxybenzoic acids** | **38.2** | **154.6** | **297.5** |
| Gallic acid *** | 8.5 | 58.8 | 189.2 |
| Syringic acid | 29.7 | 95.8 | 108.3 |
| Luteolin | - | - | 22.4 |

Delphinidin 3-glucoside is also called myrtillin and is a glucosidic anthocyanin, which is found in many green plants and is particularly abundant in blackcurrants, bilberries, cranberries, myrtle and roses. It has a red-violet colour, which together with other molecules is responsible for the coloration of the fruit.

Delphinidin 3-O-beta-D-glucoside is an anthocyanin cation consisting of delphinidin having a beta-D-glucosyl residue attached at the 3-hydroxy position and is derived from delphinidin, it is a vegetable metabolite found in several plants.

**Table 5.** Summary table of the qualitative and quantitative composition (expressed in mg/kg of total anthocyanins, total flavonols and total hydroxybenzoic acids), of the protection from cellular damage (expressed in %), polyphenols/phospholipids ratio and of the formulation efficacy (expressed in %) of the extractive vesicles according to the invention, prepared with (Et-extractive vesicles) or without ethanol (Extractive vesicles), of the corresponding extractive control solution (water soluble mixture without phospholipids with ethanol) prepared without phospholipids and with ethanol, of the phospholipidic vesicles carrying the extract obtained with the method of Perra et al. (prior art), of the phospholipidic vesicles carrying the extract obtained with the method of the patent EP3518949 A1, of the phospholipidic vesicles carrying the extract obtained with the method Manca et al. (prior art), of the phospholipidic vesicles carrying the extract obtained with the method Manconi et al. (known art).

|  | water soluble mixture without phospholipids with ethanol | extractive vesicles of the invention | Et-extractive vesicles of the invention | Extract Perra et al. in vesicles | Extract EP-3518949 A1 in vesicles | Extract EP-818225 A1 in vesicles | Manca et al. in Epithelial cells | Manconi et al. in Erythrocytes |
|---|---|---|---|---|---|---|---|---|
| **Total anthocyanins (mg/kg)** | 3429 | 6948 | 9008 | 1262 | ND | ND | 12 | 90 |
| **Total flavonols (mg/kg)** | ND | 902 | 1059 | 166 | ND | 346 | 713 | 7 |
| **Total hydroxybenzoic acids (mg/kg)** | 38 | 155 | 297 | ND | ND | 28 | 36 | 550 |
| **Protection from oxidative damage (%)** | 68 | 98 | 120 | 66 | 51 | 55 | 100 | 60 |
| **Polyphenol/phospholipid ratio** | --- | 135 | 172 | 8 | ND | ND | 4 | 4 |
| **Formulative effectiveness (%)** | | 13230 | 20460 | 528 | ND | ND | 400 | 240 |

In order to be able to compare different vesicles containing different concentrations of phospholipid and extract and with a different ability to protect cells, even of different nature, from oxidative stress, a new comparative parameter was calculated, which was indicated as formulation efficacy. This parameter is directly proportional to the concentration of total polyphenols measured in the extract divided by the concentration of phospholipid used, the constant of proportionality is given by the cell survival (%) of the cells stressed with hydrogen peroxide and protected with the formulation. The ratio of the polyphenol concentration to the phospholipid concentration indicates how many grams of polyphenols there are for each gram of phospholipid. The ratio is considerably higher in the case of extractive vesicles because these have a greater extractive power and these components extracted in greater quantities form particular vesicles with a composition and assembly different from those obtained with extraction and subsequent loading in vesicles. Multiplying by the percentage of cells that survived oxidative stress, we obtain a measure of the effective efficacy of the formulation compared to the other because we take into account the quantity of polyphenols per unit quantity of phospholipid but also the effective protective efficacy. In this case the formulation efficacy of the extractive vesicles is 4 times higher than that of Perra et al, 5 times higher than that of Manca and 8 times higher than that of Manconi. This confirms the formulation superiority of the extractive vesicles of the present invention. Furthermore, it should be considered that the extract obtained by Manca is obtained from seeds, therefore the qualitative-quantitative composition is by nature different, not comparable to the starting matrix (pomace) illustrated here.

***In vitro* permeation study**

Skin penetration and permeation studies were conducted using Franz diffusion cells with effective diffusion area of 0.785 cm$^2$ and receptor cell volume of about 5.5 ml. Between the two compartments, receptor and donor, a disk of newborn pig skin was placed with the stratum corneum facing the donor compartment; once the skin was mounted between the two compartments, held together by forceps, the receptor compartment was filled with 5.5 ml of physiological solution, taking care not to form air bubbles under the skin. 100 $\mu$l of extractive vesicle dispersion (or reference controls) were placed in the donor compartment taking care to cover the entire skin surface. For each sample, 6 cells were prepared simultaneously. The cells were thermostated at 37°C and kept under constant stirring. During the experiment, at regular intervals (every 2 hours) and up to 8 hours, all the solution of the receptor compartment was withdrawn and replaced with an equivalent volume of saline solution preheated to 37°C. The samples taken were concentrated by lyophilization, taken up with 2 ml of methanol and then analyzed in HPLC. At the end of the experiment, the skin surface was washed three times with saline and dried with filter paper. The stratum corneum was separated by stripping using Tesa® adhesive strips (Germany). The pieces of adhesive tape used for the strip were treated with methanol and subsequently sonicated in order to extract all the active substances contained in the stratum corneum adhered to the tape. The epidermis was separated from the dermis with a sterile surgical scalpel, the two layers were cut into small pieces, immersed in methanol and subsequently sonicated to ensure, also in this case, the complete extraction of the phytocomplex. The extractive solutions obtained were analyzed by HPLC. The concentration of quercetin and its derivatives was measured (Figure 1).

The Et-extractive vesicles prepared according to the invention are those which allow to obtain a greater accumulation of quercetin in the three layers of the skin (stratum corneum, epidermis and dermis) but also in the receptor compartment. The extractive vesicles according to the invention without ethanol lead to a minor accumulation of quercetin in the three layers and in the receptor compartment but still greater than that obtained using the water-soluble mixture containing ethanol without phospholipids. Therefore, the extractive vesicles of the invention allow a greater accumulation of active substances in the skin than the water-soluble mixture (without phospholipids); furthermore, by adding ethanol, the performances of the extractive vesicles further improve.

**Ability of formulations to protect skin cells from oxidative stress**

Keratinocytes were seeded in 96-well plates ($5\times10^4$ cells/well) and incubated until confluence. Then hydrogen peroxide (dilution 1 :30000) and the formulations were added to the fresh culture medium. Cells treated with hydrogen peroxide only (without extract) were used as a positive control. While untreated cells were used as a negative control (100% viability). After 4 hours of incubation, the medium was eliminated, the cells were washed with fresh culture medium and the number of remaining cells was determined with the MTT colorimetric test. Viability was calculated as a percentage of untreated cells (100% viability).

Stressful treatment with hydrogen peroxide causes 55% of cells to die and only -46% of cells to survive (Figure 2). When cells are treated simultaneously with hydrogen peroxide and Extractive vesicles cell viability increases: it is -99% when ethanol-free Extractive vesicles are used and further rises to -120% when Et-extractive vesicles are used. So the extractive vesicles completely cancel the effect of the oxidative damage, avoid cell death and even the Et-extractive vesicles promote cell proliferation. This does not occur using the extraction solution without phospholipids and containing ethanol, in this case the cell viability increases (~ 68%) compared to that of stressed and unprotected cells (~ 46%) but in any case the harmful effect of the hydrogen peroxide is not completely canceled (Figure 2).

The results obtained confirm that the extractive vesicles of our invention allow excellent protection of keratinocytes from damage caused by oxidative stress induced with hydrogen peroxide, much higher than that of the extractive solution.

**Ability of formulations to inhibit collagenase and elastase**

The anti-aging activity of the Extractive vesicles was evaluated by measuring the percentages of inhibition of collagenase and elastase. The activation of these enzymes causes the breakdown of collagen and elastin proteins, causing skin aging. In fact, these proteins form the structural matrix of the dermis and hypodermis, which gives the skin firmness and consistency. Previous studies have shown that the anti-aging activity of the extracts is directly related to their antioxidant activity and their ability to inhibit the activation of collagenase and elastase.

*Collagenase inhibition*

20 $\mu$l of enzymatic solution (0.8 units/ml), 20 $\mu$l of antioxidant samples to be tested at various dilutions and 20 $\mu$l of buffer (50 mM, pH 7.5) were poured into each well of a 96 wells plate. After 15 minutes of incubation at 37°C, 40 $\mu$l of fluorogenic reagent (MCAPro-Leu-Ala-Nva-DNP-Dap-Ala-Arg-NH$_2$, 2 mM) was added, which is enzymatically degraded by collagenase and becomes fluorescent. The dispersions were diluted with methanol (1/100), centrifuged for 2 min at 12000 rpm to remove all suspended particles, then the absorbance (ABS) was measured at 335 nm using a plate reader, at regular 20 minute intervals. Water was used as a negative control, while the extractive solution obtained according to the method reported by Perra and delivered in phospholipidic vesicles was used as a comparison. The enzymatic inhibition was calculated according to the formula:

$$\text{Collagenase inhibition (\%)} = 1 - ABS_{\text{sample}} \times ABS_{\text{water}} \times 100$$

The IC$_{50}$ value was calculated, which indicates the concentration of the sample necessary to inhibit 50% of the enzyme. The extractive vesicles of the present invention gave the highest inhibition value, IC$_{50}$=16$\pm$2 $\mu$g/ml, followed by the extractive solution prepared according to the invention but without the phospholipids therefore without the vesicles (IC$_{50}$=20$\pm$4 $\mu$g/ml), and from vesicles carrying the extract obtained according to the method reported by Perra et al. (IC$_{50}$=23$\pm$2 $\mu$g/mL).

*Elastase inhibition*

In each well of a 96 wells plate were placed 25 $\mu$l of elastase enzyme (1 g/l), 25 $\mu$l of test sample at various dilutions (1000 - 7.81 g/ml) and 25 $\mu$l of HEPES buffer (0.1 M, pH 7.5), and after 20 min at room temperature, 100 $\mu$l of reagent (N-methoxysuccinyl-AlaAla-Pro-Val-p-nitroanilide (1 mM)) were added, which releases p-nitroaniline from the substrate. The samples were left incubating for 40 min at 25°C, then they were diluted with methanol (1/100), centrifuged for 2 min at 12000 rpm to eliminate all suspended particles, finally the absorbance (ABS) was measured at 405 nm using a Tecan Infnite 500 spectrophotometer (USA). Water was used as a control. The enzymatic inhibition was calculated according to the formula:

$$\text{Elastase inhibition (\%)} = (ABS_{\text{control}}/ABS_{\text{sample}})/ABS_{\text{control}}) \times 100.$$

The extractive vesicles of the present invention gave an inhibition value IC$_{50}$=29$\pm$3 $\mu$g/ml, the extractive solution prepared according to the invention but without the phospholipids therefore without the vesicles gave IC$_{50}$=35$\pm$5 $\mu$g/ml and the vesicles carrying the extract obtained according to the method reported by Perra et al. gave IC$_{50}$=41$\pm$3 $\mu$g/ml.

**Comparison Examples**

The active substances of pomace were extracted using three different methods described in the prior art and the results were compared with those obtained with the extractive vesicles prepared following the process of the invention. With respect to what is reported in the prior art, only minimal modifications have been made in order to be able to compare the methods with each other. In this way, three different extracts were obtained which were then conveyed in the phospholipidic vesicles whose dimensions, zeta potential, antioxidant activity and ability to facilitate the penetration of the active substances into the skin were measured.

**Comparison with the extraction method reported in the article by** Perra et al in "Extraction of the antioxidant phytocomplex from wine-making by-products and sustainable loading in phospholipidic vesicles specifically tailored for skin protection" Biomedicine & Pharmacotherapy available online 29 July 2021 https: //doi.org/10.1016/j.biopha.2021.111959.

According to what reported in this work, to obtain the phospholipidic vesicles carrying the pomace extract it is necessary to carry out two separate and consecutive steps: the extraction and the delivery in the vesicles.

**Step 1) Extraction**

Solid-liquid extraction. Briefly, 50 g of grape pomace was dispersed in a mixture (250 ml) of ethanol and water (70:30 v/v, density 0.88556 g/ml). The dispersion was kept under constant stirring for 4 hours at 40°C. At the end of the extraction process, the dispersion was centrifuged twice (30 min, 8000 rpm) to separate the solid phase from the liquid one.

**Step 2) Delivery in phospholipidic vesicles**

The phospholipid (60 mg/ml of soy lecithin) was added to the extractive solution (2 ml), which was left to hydrate for one hour at room temperature. Then, the dispersion was sonicated (25 cycles 5 on 2 off) using a Soniprep 150 sonicator (MSE Crowley, London, United Kingdom).

**Characterization of the phospholipidic vesicles carrying the extract obtained according to the method reported by Perra et al.**

The mean diameter and the polydispersion index of the obtained vesicles were measured by Photon Correlation Spectroscopy, using a Zetasizer UltraNano (Malvern Instrument, UK). Before the measurement, the samples were suitably diluted (1 :100) to improve the signal quality. The zeta potential was also measured using the Zetasizer UltraNano according to the M3-PALS method (Phase Analysis Light Scattering), which measures the electrophoretic mobility of the particles inside a thermostated cuvette. Immediately after preparation, the samples were diluted and analyzed immediately (Table 6).

The unincorporated phytocomplex inside the vesicles was removed from the vesicular dispersions by dialysis. The dispersion (1 ml) was placed in a polycarbonate dialysis tube (Spectra/Por® membranes: 12-14 kDa MW cut-off, with pores 3 nm; Spectrum Laboratories Inc., USA) and dialysed in two liters of distilled water at 25°C for 2 hours, during which the water was replaced every hour. The incorporation efficiency was calculated as the percentage of phytocomplex conveyed inside the vesicles, with respect to the initial quantity used for the preparation (Table 2). The amount of the phytocomplex before and after dialysis was quantified by measuring the antioxidant activity with the DPPH (1-2,2-diphenyl-1-picrylhydrazyl) test. The antioxidant activity of the formulations was calculated according to the formula:

$$\text{Antioxidant activity \%} = [(ABS_{DPPH} - ABS_{sample}) / ABS_{DPPH}] \times 100$$

The obtained data confirm the formation of small, slightly polydisperse and negatively charged vesicles comparable to the extractive vesicles of our invention. The antioxidant activity of the vesicles carrying the extract obtained with the method reported by Perra et al. is much lower than that of the extractive vesicles of the invention. This value indicates a higher concentration of antioxidant substances in the vesicles.

Table 6. Mean diameter (MD), polydispersity index (PI), zeta potential (ZP), incorporation efficiency (EI), and antioxidant activity (AA) of vesicles incorporating pomace extract obtained by the method reported by Perra et al (Vesicles Perra et al). Mean values $\pm$ standard deviations (n=3) were reported.

|  | MD (nm) | PI | ZP (mV) | EI (%) | AA (%) |
|---|---|---|---|---|---|
| **Perra et al vesicles** | 100±4 | 0.28 | -42±5 | 76±4 | 53±2 |

The quali-quantitative composition (Table 7) of the pomace extract obtained with the method reported by Perra et al was analyzed using an Agilent 1100 HPLC consisting of a G1311A quaternary pump, a G1313A rheodyne injector, a G1316A column thermostat compartment, a G1322A degasser and a DAD UV 6000 detector (Thermo Finnigan, Milan). A Kinetex column (5u, C18, 100 A, Phenomenex, Torrance, CA, USA) and mobile phase A (acetonitrile) and B (water and 0.22 M phosphoric acid) were used.

Table 7. Quantity (mg/kg of extract) of the main components found in the extract obtained with the method reported by Perra et al..

|  | Perra et al Vesicles (mg/kg) | Extractive vesicles (mg/kg) |
|---|---|---|
| **Quercetin derivatives (360 nm)** | 166.2 | 317.4 |
| **Delphinidin and derivatives (520 nm)** | 1261.9 | 1753.1 |

Using the extraction process of Perra et al, a very poor phytocomplex is obtained wherein only quercetin and delphinidin or their derivatives are contained in concentrations that are in any case lower than those found in the extractive vesicles. Probably the other substances are present in unquantifiable concentrations and an additional concentration step of the extract would be necessary to reach quantifiable concentrations.

**Comparison with patent document EP-3518949 A1**

The EP-3518949 A1 patent describes an extraction method for lipophilic (or water insoluble) substances starting from different natural sources using a "unique one pot" process.

**Step 1) Extraction**

50 g of grape pomace was dispersed in a mixture (250 ml) of paraffin (20%), tween 80 (60%), PEG200 (10%), phospholipid (2%) and alcohol (10%). The mixture was homogenized for 30 minutes at 40°C and subsequently centrifuged at 4500 rpm for several cycles.

**Step 2) Delivery in phospholipidic vesicles**

The phospholipid (60 mg/ml of soy lecithin) was added to the extractive solution (2 ml), which was left to hydrate for one hour at room temperature. Then, the dispersion was sonicated (25 cycles 5 on 2 off) using a Soniprep 150 sonicator (MSE Crowley, London, United Kingdom). In the patent, the extract is used diluted from 2 to 98%.

**Antioxidant activity and quali-quantitative composition**

The antioxidant activity of the phytocomplex conveyed in the vesicles was measured with the DPPH (1-2,2-diphenyl-1-picrylhydrazyl) test. In this case the antioxidant activity of the pomace extract obtained with the method reported in the patent EP-3518949 A1 **and conveyed in the vesicles** is very low, 23±4% (Table 8). The result obtained indicates that not many antioxidant substances have been extracted as occurs instead using the extractive vesicles of our invention, whose antioxidant activity measured under the same conditions is 78-82% (Table 8).

The polyphenol content was determined using an Agilent 1100 HPLC consisting of a G1311A quaternary pump, a G1313A rheodyne injector, a G1316A column thermostat compartment, a G1322A degasser, and a DAD UV 6000 detector (Thermo Finnigan, Milan). The analyzes were performed using a Kinetex column (5u, C18, 100 A, Phenomenex, Torrance, CA, USA) and as mobile phase A (acetonitrile) and B (water and 0.22 M phosphoric acid). No active component was detected in the extract, therefore the method is not suitable for the extraction of the antioxidant phytocomplex from pomace.

**Comparison with patent document EP-818225 A1**

To compare the extraction process of EP-818225 A1 **patent with that of our invention,** the extraction method III reported in the descriptive part was used. In this patent a lipophilic extract enriched with lycopene has been obtained starting from dry peels of *Lycopersicum esculentum.*

**Step 1) Extraction**

50 g of pomace were dispersed in 250 ml of water and left to rest for one hour, thus allowing the vegetable material swelling, cell rupture and membrane porosity. The dispersion was mixed with 800 ml of normal-hexane containing 0.01% soy lecithin and kept under stirring for 3 hours. Subsequently, the normal-hexane was separated by sedimentation, and the residue was treated again with 400 ml of normal-hexane. The solutions of normal-hexane were combined, filtered and the solvent was completely removed under vacuum, thus obtaining an oil which, as described by the same patent, can be used as such on the skin (traces of normal-hexane may still be present, which is toxic to the skin).

**Step 2) Delivery in phospholipidic vesicles**

The phospholipid (120 mg of soy lecithin) was mixed with 0.6 mg of oil extract (it was not possible to use a higher amount). The lipids were hydrated with 2 ml of water, propylene glycol, glycerol and ethanol and left for one hour at room temperature.Then, the dispersion was sonicated (25 cycles 5 on 2 off) using a Soniprep 150 sonicator (MSE Crowley, London, United Kingdom).

**Antioxidant activity**

The antioxidant activity of the phytocomplex obtained with the method reported in the patent EP-818225 A1 and conveyed in the vesicles is 55±4%, therefore much lower than that of the extractive vesicles of our invention.

**Quali-quantitative characterization of the extract**

The components of the extract were identified and quantified using an Agilent HPLC (Table 8). The only components identified in the extract are quercetin, its derivatives and luteolin, their quantity is comparable to that found in the extractive vesicles. The fundamental difference lies in the fact that in the extractive vesicles there are 14 additional components (Table 8), in higher quantities, while in the extract obtained according to the patent document EP-818225 A1, no other antioxidant molecules have been identified. Therefore, the extract is certainly poorer in quantity and quality than that obtained with the extractive vesicles of our invention.

**Table** 8. Quantity (mg/kg of extract) of the main components found in the extract obtained with method III reported in the patent document EP-818225 A1.

| Compounds | Et-extractive vesicles | Hydro soluble mixture EP-818225 A1 |
|---|---|---|
| | mg/kg | mg/kg |
| **Total anthocyanins** | **9008.4** | - |
| **Delphinidine derivatives** | 398.2 | |
| **Delphinidine derivatives** | 971.3 | |
| **Delphinidin 3 glucoside** | 1127.5 | |
| **Delfinidin-3-acetylglucoside** | 438.9 | |
| **Delphinidine derivatives** | 291.9 | |
| **Delphinidine derivatives** | 296.4 | |
| **Delphinidine derivatives** | 693.0 | |
| **Delphinidine derivatives** | 482.3 | |
| **Peonidin-3-glucoside** | 567.1 | |
| **Malvidin-3-acetylglucoside** | 554.3 | |
| **Malvidin-3- glucoside** | 3187.5 | |
| **Flavonoids** | **mg/kg** | **mg/kg** |
| **Total Flavonoids** | **1058.9** | **343.05** |
| **Iso-quercetin** | 245.1 | 196.60 |
| **Quercetin derivative** | 317.4 | 49.23 |
| **Quercetin Glucopyranoside** | 496.4 | 97.22 |
| **Hydroxybenzoic acids** | **mg/kg** | **mg/kg** |
| **Total hydroxybenzoic acids** | **297.5** | **27.84** |
| **Gallic acid** | 189.2 | |
| **Syringic acid** | 108.3 | |
| **Luteolin** | 22.4 | 27.84 |

**Summary of the data obtained**

As regards the extraction efficiency of the extractive vesicles of our invention, the results of the qualitative-quantitative composition of our extract are much higher than those obtained using the method reported in the article by Perra et al or in the two patents (summary Table 9). The extract contained in the Extractive vesicles (with and without ethanol) has a much higher antioxidant activity and a much richer qualitative-quantitative composition (Table 3).

**Table 9.** Quali-quantitative composition of the phytocomplexes obtained using the different extraction methods: in water, propylene glycol, glycerol and ethanol (a water-soluble mixture like our invention but without phospholipids); in water, propylene glycol, glycerol, ethanol and phospholipids (Et-extractive vesicles of our invention); in water, ethanol at 40°C (Perra et al); in lipids according to method II of patent EP3518949 A1; in normal-hexane according to method III of patent EP-818225 A1.

| Compounds | Water-soluble mixture without phospholi pids with ethanol | Et-extractive vesicles of the invention | Water-soluble mixture Perra et al. | Water soluble mixture EP-351894 9 A1 | Water soluble mixture EP-818225 A1 |
|---|---|---|---|---|---|
| **Anthocyanins *** | **mg/kg** | **mg/kg** | **mg/kg** | **mg/kg** | **mg/kg** |
| | 203.5 | 398.2 | | | |
| | 701.6 | 971.3 | | | |
| Delphinidin 3 glucoside | 497.6 | 1127.60 | 371.22 | | |
| Delphinidin 2 glucoside | 249.7 | 438.9 | 452.14 | | |
| Delphinidin 1 glucoside | 105.8 | 291.9 | 438.62 | | |
| | 189.3 | 296.4. | | | |
| | 154.9 | 693.0 | | | |
| | NO | 482.3 | | | |
| | 169.9 | 567.1 | | | |
| | 144.9 | 554.30 | | | |
| | 1011.5 | 3187.5 | | | |
| **Flavonols** | **mg/kg** | **mg/kg** | **mg/kg** | **mg/kg** | **mg/kg** |
| Iso-quercetin | 60.6 | 245.1 | | | 196.60 |
| Quercetin derivative ** | 124.4 | 317.4 | 166.17 | | 49.23 |
| Quercetin Glucopyran oside | 183.6 | 496.4 | | | 97.22 |
| **Hydroxybenzoic acids** | **mg/kg** | **mg/kg** | **mg/kg** | **mg/kg** | **mg/kg** |
| Gallic acid *** | 8.5 | 189.2 | | | |
| Syringic acid | 29.7 | 108.3 | | | |
| Luteolin | | 22.4 | | | 27.84 |

As regards **the ability to deliver the active substances into the skin,** the extractive vesicles of our invention allow to reach the highest accumulation of quercetin in the three layers of the skin. The highest values were obtained with the Et-extractive vesicles (Figure 1). By conveying the extract obtained with the method of Perra et al or with that of the patents indicated in table 9 in the same phospholipidic vesicles, the cutaneous penetration of quercetin is much lower and always less than 1%, in all three layers of the skin. This confirms the better cutaneous delivery capacity of the extractive vesicles, wherein the phospholipids of the vesicular bilayer are probably intercalated by the various components of the phytocomplex, modifying its packing and probably making it more fluid and more suitable for interacting with the skin. Furthermore, glycerol and propylene glycol are promoters of skin absorption and exert a synergistic action with the vesicles in favoring the penetration of bioactive substances. Thus, the extractive vesicles of the present invention can be added to lotions, serums, gels, creams, pastes, scrubs, detergents, soaps, sprays and foams to be applied on the skin or even on the mucous membranes to increase the delivery of the active substances and improve their local effectiveness. Due to their composition, they can be used on even the most delicate and sensitive skin of newborns, children and the elderly.

Regarding the ability of the formulations to protect skin cells from oxidative stress, the stressful treatment with hydrogen peroxide, as mentioned, causes ~55% of the cells to die and only ~46% of the cells to survive (Figure 4). When cells are treated simultaneously with hydrogen peroxide and Extractive vesicles cell viability is ~99% when ethanol-free vesicles are used and further rises to ~120% when Et-extractive vesicles are used. So the extractive vesicles completely cancel the effect of the oxidative damage, avoid cell death and the Et-extractive vesicles promote even cell proliferation. This does not occur using the extractive solution without phospholipids and containing ethanol and the other vesicles obtained by means of the prior art and the patents under examination. In this case the cell viability increases by ~ 68% for the water-soluble mixture with ethanol, while by conveying the extract obtained with the method of Perra et al or with that of the patents in the same phospholipidic vesicles, the viability is respectively ~ 65%. ~51% and -55% compared to that of stressed and unprotected cells (-46%), thus they are unable to completely reverse the damaging effect of hydrogen peroxide (Figure 2).

The extractive vesicles of the present invention can be used for the extraction of the antioxidant phytocomplex from vegetable biomass and to facilitate their administration both topically on the skin or mucous membranes and orally to obtain an effect both at an intestinal and systemic level. Topically they can also be used on children and the elderly as they are highly biocompatible and biodegradable.

**Example of anti-aging gel or serum:**

Hydrolysed collagen 1-2% (w/v)
Hyaluronic acid 1-2% (w/v)
Antibacterials 0.01-0.1% (w/v)
Perfume (0-0.5% v/v)
Et- Extractive vesicles q.b to 100 ml

Formulation of an elasticising nourishing cream:

Argan oil
Montanov® (1-5% w/v)
Vitamin E acetate (0.1-1% w/v)
Sepigel 305® (0.5-1.5% w/v)
Antibacterials 0.01-0.1% (w/v)
Perfume (0-0.5% v/v)
Et- Extractive vesicles q.b to 100 ml

NUTRACEUTICAL EXAMPLE OBTAINED FROM ARTICHOKE WASTE

The artichoke cultivation waste (50-100 g) was dispersed in a mixture containing water, glycerol, propylene glycol and ethanol. Soy lecithin was added to the dispersion and after 1 hour, the dispersion was vigorously stirred by sonication (99 cycles 5 on 2 off, 13 micron probe amplitude), using a Soniprep 150 sonicator (MSE Crowley, London, United Kingdom) to aid extraction and vesicle formation. Then the dispersion was kept under continuous stirring for 3 hours at 45°C. At the end of the process, the dispersion was centrifuged three times (30 min, 8000 rpm) to separate the exhausted biomass from the aqueous dispersion containing the vesicles carrying the phytocomplex.

**Composition**

A mixture containing water (65% v/v), propylene glycol (5% v/v), glycerol (20% v/v) and ethanol (10% v/v) was selected as extractive solution and vesicle hydrating medium.. The quantities of artichoke waste (between 100 and 200 mg/ml) and soy lecithin (60-120 mg/ml) were varied to obtain stable systems containing a richer and more concentrated phytocomplex.

With the aim of developing a nutraceutical formulation, a polymer (10 mg/ml of sodium alginate) was added to the formulation of the extractive vesicles (2 ml). Then, the dispersion was sonicated (35 cycles 5 on 2 off) using a Soniprep 150 sonicator (MSE Crowley, London, United Kingdom).

**Characterization of phospholipidic vesicles**

The mean diameter and the polydispersion index of the obtained vesicles were measured by Photon Correlation Spectroscopy, using a Zetasizer UltraNano (Malvern Instrument, UK). Before the measurement, the samples were suitably diluted (1 :100) to improve the signal quality. The zeta potential was also measured using the Zetasizer UltraNano according to the M3-PALS analysis (Phase Analysis Light Scattering), which measures the electrophoretic mobility of the particles inside a thermostated cuvette. Immediately after preparation, the samples were diluted and analyzed immediately (Table 10).

**Table 10.** Mean diameter (MD), polydispersion index (PI), zeta potential (ZP), and incorporation efficiency (EI) of Extractive vesicles with and without sodium alginate. Mean values $\pm$ standard deviations (n$\geq$3) were reported.

|  | MD (nm) | PI | ZP (mV) | EI (%) |
|---|---|---|---|---|
| Extractive vesicles | 145$\pm$2 | 0.27 | -44$\pm$5 | 92$\pm$4 |
| Alginate_Extractive vesicles | 165 | 0.31 | -49$\pm$3 | 93$\pm$2 |

The phytocomplex not incorporated inside the vesicles was removed from the vesicular dispersions by dialysis. Each dispersion (1 ml) was placed in a polycarbonate dialysis tube (Spectra/Por® membranes: 12-14 kDa MW cut-off, with pores 3 nm; Spectrum Laboratories Inc., USA) and dialysed in two liters of distilled water at 25°C for 2 hours, during which the water was replaced every hour. The incorporation efficiency (EI) was calculated as the percentage of phytocomplex conveyed inside the vesicles, with respect to the initial quantity used for the preparation. The amount of the phytocomplex before and after dialysis was quantified by measuring the antioxidant activity with the DPPH (1-2,2-diphenyl-1-picrylhydrazyl) test. The antioxidant activity of the formulations was calculated according to the formula:

$$AA\% = [(ABS_{DPPH} - ABS_{sample}) / ABS_{DPPH}] \times 100.$$

**Antioxidant activity of the formulations**

The antioxidant activity of vesicles prepared with polymer (Alginate-extractive vesicles) and without (extractive vesicles) was measured and used to make a first evaluation of the extraction efficacy of the systems. As a comparison, water-soluble mixtures having the same composition as the extractive vesicles but without the addition of phospholipids were used.

**Table 11.** Antioxidant activity of the extraction solution without phospholipids and without sodium alginate, used as a comparison, and of the Extractive vesicles prepared with sodium alginate (Alginate-extractive vesicles) and without sodium alginate (extractive vesicles). Mean values $\pm$ standard deviations (n=6) were reported.

|  | Antioxidant activity (%) | Soy lecithin | Sodium Alginate |
|---|---|---|---|
| Water soluble mixture | 59$\pm$2 | no | no |
| Extractive vesicles | 84$\pm$2 | yes | no |
| Alginate extractive vesicles | 83$\pm$3 | yes | yes |

The antioxidant activity of the water soluble blend is -59%. The antioxidant activity of the extractive vesicles obtained according to the invention and not containing sodium alginate is -84% and does not change for those containing the polymer, in fact it is -83%. The results confirm a better antioxidant activity of the extractive vesicles of the invention compared to the corresponding extractive solutions without phospholipids, which indicates, also in this case, a greater extraction of substances with an antioxidant character.

**Quali-quantitative analysis**

Quali-quantitative analysis was performed using an Agilent 1100 HPLC consisting of a G1311A quaternary pump, a G1313A rheodyne injector, a G1316A column thermostat compartment, a G1322A degasser and a DAD UV 6000 detector (Thermo Finnigan, Milan).
The analysis confirms that the extractive vesicles of the invention allow to extract a greater quantity of active substances and in higher quantities (Table 12). The main active substances found in the extractive vesicles are caffeic acid, chlorogenic acid and its derivatives, luteolin and derivatives and apigenin.

**Table 12.** Quantity (mg/kg of extract) of the main antioxidant components found in the extractive vesicles and in the corresponding water-soluble mixture. Mean values ± standard deviations (n=3) were reported.

| Compounds | Water soluble mixture without phospholipids | Extractive vesicles |
|---|---|---|
| | **mg/kg** | **mg/kg** |
| **Total polyphenols** | 701.6 | 1326.3 |
| **Total caffeoylquinic acids** | **198.6** | 395.5 |
| 1-O-caffeoylquinic acid | - | 42.0 |
| 3-O-caffeoylquinic acid | 23.7 | 37.1 |
| 5-O-caffeoylquinic acid (chlorogenic acid) | 78.6 | 132.9 |
| Caffeic acid | 9.3 | 14.1 |
| 1 ,5-di-O-caffeoylquinic acid (cynarin) | - | 12.3 |
| Other di-caffeoylquinic acids | 87.0 | 157.0 |
| **Total flavonoids** | 503.0 | 930.8 |
| Luteolin 7 - glucoside | 202.4 | 403.1 |
| Luteolin | 300.6 | 426.2 |
| Apigenin | - | 101.4 |

**Stability studies in acid and basic environments.**

Oral administration involves the passage of the preparation through the gastro-intestinal tract, which has an acidic pH in the stomach and neutral in the intestine. For this reason, the stability of the extractive vesicles was evaluated in an acidic and high ionic strength solution, which simulates the gastric environment and in a neutral pH and high ionic strength solution, which simulates the intestinal environment (Table 13). The dimensions of the extractive vesicles as well as their polydispersion index increase significantly immediately after dilution with the pH 2 solution and the zeta potential decreases considerably, almost completely canceling the surface charge, becoming positive in some cases. The presence of sodium alginate, on the other hand, seems to stabilize the vesicles, whose dimensions and polydispersion index do not undergo significant variations, while the surface charge also in this case decreases or reverses.

At pH 7, the average dimensions and the polydispersion index of the extractive vesicles with and without alginate increase slightly, while the zeta potential decreases but still remains negative, with no significant differences between the different formulations. At neutral pH the vesicles are more stable and the sodium alginate is able to better protect the vesicular structure ensuring greater protection of the phytocomplex.

**Table 13**: Mean diameter (DM), polydispersion index (PI) and zeta potential of Extractive vesicles with and without sodium alginate diluted with a pH 2 solution and with a pH 7 solution and kept at 37°C for 2 hours at pH2 and/or 6 hours at pH 7.

| | Time | DM (nm) | | PI | | Zeta potential (mV) | |
|---|---|---|---|---|---|---|---|
| | | pH 2 | pH 7 | pH 2 | pH 7 | pH 2 | pH 7 |
| Extractive vesicles | t0 | 1099±42 | 204±10 | 0,51 | 0,26 | 0±, | -17±1 |
| | t2/6h | 2303±27 | 223±27 | 0,76 | 0,31 | 3±, | -7±3 |
| Alginate Extractive vesicles | t0 | 178±31 | 182±9 | 0,30 | 0,14 | -0±2 | -10±2 |
| | t2/6h | 254±28 | 186±6 | 0,32 | 0,18 | 5±2 | -5±1 |

**CONCLUSIONS**

The extraction carried out simultaneously with the assembly of the extractive vesicles is the key process which makes the extractive vesicles of the invention unique because it allows to extract a greater number of functional substances with respect to the prior art and to efficiently intercalate them in the lipid bilayer instead of only in the hydrophilic vehicle contrary to what happens in the known art when it is extracted using only the mixture of aqueous solvents. The formation of the vesicles at the same time as the extraction allows to extract a greater quantity of functional substances from the biomasses and, at the same time, allows these molecules to intercalate perfectly in the phospholipid bilayer, influencing its assembly and allowing a closer interaction between phospholipids and functional substances, which makes these vesicles unique for their composition and richness in flavonols and anthocyanins, as reported in Table 4 When the extraction is done using only an aqueous extractive mixture (without phospholipids), the resulting phytocomplex lacks a part of the functional substances, the less polar ones, as confirmed by the results obtained with only the extractive mixture without phospholipids or with the extractive mixtures of the known art (see for instance Perra, Manca, Manconi). Clearly, if the partial phytocomplex is subsequently loaded into the vesicles, these will be different because the loaded substances are quite soluble in the extractive mixture and therefore tend to remain in solution and not to intercalate between the phospholipids. In order for these to intercalate between the phospholipids, it is necessary to greatly increase the concentration of extract in the dispersion. However, the interaction of phospholipids with these partially polar molecules is different from that which occurs in the extractive vesicles of the invention. For this reason the extractive vesicles have a very low ratio (0.15) between the concentration of phospholipid and concentration of extract, while the vesicles prepared according to the prior art using the previously separated, dried and rehydrated extract, the ratio between concentration of phospholipid and concentration of extract is very high (3-5) because it is necessary to load a lot of extract to obtain a relevant biological effect.

The advantage of the low ratio between phospholipid concentration and extract concentration is given by the fact that for each single molecule of functional substance there are more phospholipid molecules which facilitate its crossing of biological membranes while molecules alone without phospholipids cannot easily cross biological membranes. Furthermore, the hydrophilic-lipophilic extraction system used is highly specific for the delivery in the human body, because it is formed by a phospholipid bilayer, the same that constitutes cell membranes. Therefore, the extractive system according to the invention has a structure and composition comparable to that of biological tissues wherein the active substances must go to exert their beneficial effects. In confirmation of this, the extractive vesicles allow to protect the target cells in a superior way compared to the vesicles of the prior art, as reported in Table 5.

For this same reason the extractive vesicles facilitate the accumulation of the functional substances conveyed in the skin in a superior way compared to the other systems, as can be seen from Fig. 3e

**Table 14:** Concentration (%) of quercetin accumulated in the stratum corneum, epidermis and dermis or permeated into the receptor compartment after 8 hours of application of the Extractive vesicles according to the invention, prepared with (Et-extractive vesicles) or without ethanol (extractive vesicles) or of the corresponding extractive control solution (water soluble mixture without phospholipids with ethanol) prepared without phospholipids and with ethanol, of the phospholipidic vesicles carrying the extract obtained with the method of Perra et al. (prior art), phospholipidic vesicles carrying the extract obtained with the method of the patent EP3518949 A1 and phospholipidic vesicles carrying the extract obtained with that of the patent EP-818225 A1.

| Accumulated quercetin (%) | Water soluble mixture without phospholipids with ethanol | Extractive vesicles of the invention | Et-extractive vesicles of the invention | Extract Perra et al. In vesicles | Extract EP-3518949 A1 in vesicles | Extract EP-818225 A1 in vesicles |
|---|---|---|---|---|---|---|
| STRATUM CORNEUM | 8.11% | 12% | 17.55% | 5.28% | 2.69% | 1.84% |
| EPIDERMIS | 5.08% | 8.9% | 8.57% | 2.24% | 0.35% | 0% |
| DERMIS | 0.20% | 0.43% | 0.45% | 0.43% | 0.39% | 0% |
| RECEPTOR | 1.00% | 1.17% | 4.08% | 1.13% | 0.26% | 0.26% |

Compared to the three works cited, the article by Perra et al. was taken into greater consideration as the study has the same final objective, namely that of formulating and characterizing phospholipidic vesicles carrying a pomace extract for topical application.

The Perra et al. method is carried out at least in two separate and consecutive steps: extraction and vehiculation in the vesicles, integrated by an intermediate stage of freeze-drying of the extract, which is then rehydrated to form the vesicles. After the extraction, Perra's method must necessarily carry out a freeze-drying process that allows him to obtain a concentrated extract rich in bioactives.

The method presented in the invention allows, as mentioned, to extract and convey the phytocomplex contained in the vegetable material in a single step, through the addition of phospholipids (soy lecithin) to the aqueous extractive solution. Phospholipids increase extraction efficiency and allow delivery in nanocarriers. The process, thanks to the addition of phospholipids, avoids the use of toxic organic solvents, in any case obtaining a phytocomplex rich in active substances and already incorporated in phospholipidic vesicles. The latter are able to increase the accumulation, bioavailability and effectiveness of the phytocomplex on the skin (green, one-shot and ready-to-use process).

**Claims**

1. Phospholipidic vesicles obtained by extraction from raw products of vegetable processing wherein the extraction, the loading of the phytocomplex and the formation of the vesicles are obtained simultaneously in a single stage by adding to said raw products:

   - a water-soluble mixture containing water in quantities ≥ 50% v/v, one or more food grade glycols in quantities 1-30% v/v and ethanol in quantities 0-20% v/v; and
   - one or more phospholipids in an amount of 6-9% w/v with respect to said water-soluble mixture.

2. The phospholipidic vesicles according to claim 1 wherein the one or more glycols are selected from: propylene glycol, ethylene glycol, polyethylene glycol, glycerol.

3. The phospholipidic vesicles according to any one of claims 1-2 wherein the one or more phospholipids are selected from among phosphatidylcholine, phosphatidyl-ethanolamine, phosphatidyl-glycerol, phosphatidyl-serine, phosphatidyl-inositol, dimyristoyl-phosphatidyl-choline, dipalmitoylphosphatidyl-choline, distearoyl-phosphatidyl-choline, palmitoyl-stearoyl-phosphatidyl-choline, sphingomyelin, such as soy or egg lecithins, hydrogenated or non-hydrogenated, their derivatives, their mixtures; alone or mixed with one or more lipids selected from: cholesterol, phytosterol, oleic acid, linoleic acid, squalene and carotenes.

4. The phospholipidic vesicles according to any one of claims 1-3 wherein the raw products of vegetable processing are selected from pomace and artichoke, tomato and olive processing waste, preferably pomace obtained after racking off and pomace deriving from distillation processes.

5. The phospholipidic vesicles according to any one of claims 1-4 wherein the raw products comprise one or more of the following components: seeds, leaves, fruits, flowers, branches, stalks, roots, bark.

6. The phospholipidic vesicles according to any one of claims 1-5 in dehydrated form, or dehydrated and rehydratable in contact with an aqueous environment.

7. Compositions comprising the phospholipidic vesicles according to any one of claims 1-6 and at least one of nutraceutically acceptable components, pharmaceutically acceptable excipients, cosmetically acceptable excipients, pH regulators, preservatives, hyaluronic acid alone or in combination with chelating agents and preservatives, allantoin, ellagic acid, chondroitin and mixtures thereof.

8. The compositions according to the preceding claim formulated for oral or topical administration on the skin and mucous membranes.

9. The phospholipidic vesicles or compositions according to any one of the preceding claims for use in the prevention and treatment of conditions or affections which benefit from the administration of antioxidant substances present in said raw materials.

10. The phospholipidic vesicles or compositions according to any one of claims 1-8 for use in the treatment of oxidative stress.

11. The phospholipidic vesicles or compositions according to any one of claims 1-8 for use in the prevention and treatment of scarring, keloids, atopic and seborrheic dermatitis, psoriasis.

12. Cosmetic use of phospholipidic vesicles or compositions according to any one of claims from 1 to 8 for the anti-aging treatment of the skin, with antioxidant, draining, moisturizing and nourishing activity or with a post-peeling soothing function of the living being, human or animal, including the elderly, children and infants.

13. A process for the production of phospholipidic vesicles comprising the basic steps of:

i. Preparing a water-soluble mixture containing water in an amount greater than 50%, preferably 50-98%v/v, one or more food grade glycols, such as for instance propylene glycol and/or polyethylene glycol and/or glycerol in an amount of 1-30 % v/v, ethanol in an amount of 0-20% v/v;
ii. Preparing a hydrated biomass starting from vegetable material processing waste whose water content is about 2-20% w/v based on the solid material of the biomass;
iii. Adding to the hydrated biomass an extractive mixture in a quantity of 2-30% w/v with respect to the biomass so as to obtain a mixture being treated, said extractive mixture being constituted by 100% of the water-soluble mixture of stage i.) to which 6-9% w/v of one or more phospholipids with respect to said water-soluble mixture are added;
iv. Letting the mixture being treated of step iii.) hydrate at room temperature for about 0.5-2 hours;
v. Stirring the mixture of the previous stage for 1-4 hours at 30-50°C, to favor the extraction of the phytocomplex and the formation of vesicles;
vi. Separating the exhausted biomasses from the dispersion containing the extractive vesicles, for instance, by centrifugation or other similar method and recovering the extractive vesicles;

**vii.** Possibly purifying the vesicles, for instance by dialysis.

14. The process according to the preceding claim wherein said extractive mixture consists of 100% of the water-soluble mixture to which is added 6-9% w/v of one or more phospholipids based on said water-soluble mixture.

15. An extractive mixture consisting of a water-soluble mixture comprising water in an amount greater than 50%, preferably 50-98% v/v, one or more food grade glycols, such as for instance propylene glycol and/or polyethylene glycol and/or glycerol in an amount of 1 -30% v/v, ethanol in an amount of 0-20% v/v, said extractive mixture being constituted by 100% of the water-soluble mixture to which is added 6-9% w/v of one or more phospholipids based on said water soluble mixture.

Fig. 1

Fig. 2

Europäisches
Patentamt

European
Patent Office

Office européen
des brevets

# EUROPEAN SEARCH REPORT

Application Number

EP 23 18 7122

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PERRA MATTEO ET AL: "Extraction of the antioxidant phytocomplex from wine-making by-products and sustainable loading in phospholipid vesicles specifically tailored for skin protection", BIOMEDICINE & PHARMACOTHERAPY, ELSEVIER, FR, vol. 142, 29 July 2021 (2021-07-29), XP086785438, ISSN: 0753-3322, DOI: 10.1016/J.BIOPHA.2021.111959 [retrieved on 2021-07-29] | 1,7,9, 10,12,15 | INV. A23L5/00 A61K8/31 A61K36/00 A61K47/46 A61Q19/00 |
| Y | * the whole document * * paragraphs [02.3], [02.6] * * page 10, left-hand column * | 1-12 | |
| X | MANCA MARIA LETIZIA ET AL: "From waste to health: sustainable exploitation of grape pomace seed extract to manufacture antioxidant, regenerative and prebiotic nanovesicles within circular economy", SCIENTIFIC REPORTS , vol. 10, no. 1 1 January 2020 (2020-01-01), XP093023890, DOI: 10.1038/s41598-020-71191-8 Retrieved from the Internet: URL:https://www.ncbi.nlm.nih.gov/pmc/articles/PMC7447760/pdf/41598_2020_Article_71191.pdf | 1,7,9, 10,12,15 | |
| Y | * the whole document * * page 3 * | 1-12 | TECHNICAL FIELDS SEARCHED (IPC) A23L A61K A61Q |

-/--

The present search report has been drawn up for all claims

| Place of search | Date of completion of the search | Examiner |
|---|---|---|
| Munich | 13 November 2023 | Orlando, Michele |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons

& : member of the same patent family, corresponding
 document

EPO FORM 1503 03.82 (P04C01)

page 1 of 2

Europäisches
Patentamt

European
Patent Office

Office européen
des brevets

## EUROPEAN SEARCH REPORT

Application Number

EP 23 18 7122

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MANCONI MARIA ET AL: "Nano-incorporation of bioactive compounds from red grape pomaces:In vitro and ex-vivo evaluation of antioxidant activity", INTERNATIONAL JOURNAL OF PHARMACEUTICS, ELSEVIER, NL, vol. 523, no. 1, 20 March 2017 (2017-03-20), pages 159-166, XP029977516, ISSN: 0378-5173, DOI: 10.1016/J.IJPHARM.2017.03.037 | 1,7,9, 10,12,15 | |
| Y | * the whole document * <br> * paragraphs [02.2], [02.3], [03.2] * <br> * page 165, right-hand column * <br> ----- | 1-12 | |
| A | EP 0 818 225 A1 (INDENA SPA [IT]) 14 January 1998 (1998-01-14) * the whole document * ----- | 1-15 | |
| A | EP 3 518 949 A1 (YISSUM RES DEV CO OF HEBREW UNIV JERUSALEM LTD [IL]) 7 August 2019 (2019-08-07) * the whole document * * claims; examples * ----- | 1-15 | TECHNICAL FIELDS SEARCHED (IPC) |

The present search report has been drawn up for all claims

| Place of search | Date of completion of the search | Examiner |
|---|---|---|
| Munich | 13 November 2023 | Orlando, Michele |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 18 7122

This annex lists the patent family members relating to the patent documents cited in the above-mentioned European search report.
The members are as contained in the European Patent Office EDP file on
The European Patent Office is in no way liable for these particulars which are merely given for the purpose of information.

13-11-2023

| Patent document cited in search report | | Publication date | Patent family member(s) | | Publication date |
|---|---|---|---|---|---|
| EP 0818225 | A1 | 14-01-1998 | AT | E227599 T1 | 15-11-2002 |
| | | | AU | 722774 B2 | 10-08-2000 |
| | | | CA | 2210039 A1 | 12-01-1998 |
| | | | DE | 69717015 T2 | 09-10-2003 |
| | | | DK | 0818225 T3 | 10-03-2003 |
| | | | EP | 0818225 A1 | 14-01-1998 |
| | | | ES | 2186824 T3 | 16-05-2003 |
| | | | HK | 1005499 A1 | 15-01-1999 |
| | | | IT | MI961442 A1 | 12-01-1998 |
| | | | JP | 4338232 B2 | 07-10-2009 |
| | | | JP | H10226654 A | 25-08-1998 |
| | | | KR | 980008222 A | 30-04-1998 |
| | | | PT | 818225 E | 31-03-2003 |
| | | | US | 5897866 A | 27-04-1999 |
| EP 3518949 | A1 | 07-08-2019 | EP | 3518949 A1 | 07-08-2019 |
| | | | US | 2019314432 A1 | 17-10-2019 |
| | | | US | 2022088099 A1 | 24-03-2022 |
| | | | WO | 2018061011 A1 | 05-04-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 818225 A
- EP 3518949 A1

- EP 818225 A1

### Non-patent literature cited in the description

- **PERRA M. et al.** Extraction of the antioxidant phytocomplex from winemaking by-products and sustainable loading in phospholipidic vesicles specifically tailored for skin protection. *Biomedicine & Pharmacotherapy,* 29 July 2021, https://doi.org/10.1016/j.biopha.2021.111959
- **MANCA M. et al.** From waste to health: sustainable exploitation of grape pomace seed extract to manufacture antioxidant, regenerative and prebiotic nanovesicles within circular economy. *SCIENTIFIC REPORTS,* 01 January 2020, vol. 10 (1
- Nano-incorporation of bioactive compounds from red grape pomace: In vitro and ex-vivo evaluation of antioxidant activity. **MANCONI M. et al.** INTERNATIONAL JOURNAL OF PHARMACEUTICS. ELSEVIER, 20 March 2017, vol. 523, 159-166

- **C. PENG ; S. CHEN ; F. LIN ; Z. LIN.** Detection of antioxidative capacity in plants by scavenging organic free radical DPPH Progress. *Biochemistry and Biophysics,* 2000, vol. 27 (6), 57-61
- **M. CHI ; C. ZHANG ; G. ZHENG ; X. MEI.** Determination of radical scavenger in Chinese drug by spectrophotometry. *China Journal of Traditional Chinese Medicine and Pharmacy,* 2003, vol. 18 (9), 567-568
- **O. OZCELIK ; J.H. LEE ; D.B. MIN.** Effects of light, oxygen and pH on the absorbance of 2,2-diphenyl-1-picrylhydrazyl. *Journal of Food Science,* 2003, vol. 68, 487-490
- **PERRA et al.** Extraction of the antioxidant phytocomplex from wine-making by-products and sustainable loading in phospholipidic vesicles specifically tailored for skin protection. *Biomedicine & Pharmacotherapy,* 29 July 2021, https: //doi.org/10.1016/j.biopha.2021.111959